# EUROPEAN PATENT SPECIFICATION

(11) **EP 4 499 208 B1**
(45) Date of publication and mention of the grant of the patent: **29.04.2026**
(21) Application number: 23714099.1
(22) Date of filing: 13.03.2023
(51) Int. Cl.: A61N 1/365, A61N 1/372, A61N 1/375

(54) **APPARATUS FOR ATRIAL SYNCHRONOUS VENTRICULAR PACING**
VORRICHTUNG FÜR ATRIALSYNCHRONE VENTRIKULÄRE STIMULATION
APPAREIL DE STIMULATION VENTRICULAIRE SYNCHRONE AURICULAIRE

(30) Priority: 31.03.2022 US 202263325864 P
(43) Date of publication of application: 05.02.2025
(73) Proprietor: Medtronic, Inc., Minneapolis, Minnesota 55432 (US)
(72) Inventor: SHELDON, Todd J., Minneapolis, Minnesota 55432 (US); PAUL, Alyssa Lauren, Minneapolis, Minnesota 55432 (US)
(74) Representative: Zimmermann & Partner Patentanwälte mbB
(86) International application number: PCT/IB2023/052403
(87) International publication number: WO 2023/187521

(56) References cited:
- US-A1- 2020 147 396
- US-A1- 2020 179 708
- US-A1- 2021 236 825
- HEIST E. KEVIN: "Atrioventricular Synchronous Leadless Pacing", JACC: CLINICAL ELECTROPHYSIOLOGY, vol. 6, no. 1, 1 January 2020 (2020-01-01), US, pages 107 - 110, XP093049215, ISSN: 2405-500X, Retrieved from the Internet <URL:https://www.sciencedirect.com/science/article/pii/S2405500X19308448/pdfft?md5=224edda3bcf28045258b3567ce0ba92a&pid=1-s2.0-S2405500X19308448-main.pdf> [retrieved on 20230523], DOI: 10.1016/j.jacep.2019.11.001

## Description

### TECHNICAL FIELD

This disclosure relates to a medical device and method for delivering atrial synchronous ventricular pacing.

### BACKGROUND

During normal sinus rhythm (NSR), the heartbeat is regulated by electrical signals produced by the sino-atrial (SA) node located in the right atrial wall. Each intrinsic atrial depolarization signal produced by the SA node spreads across the atria, causing the depolarization and contraction of the atria, and arrives at the atrioventricular (AV) node. The AV node responds by propagating a ventricular depolarization signal through the Bundle of His (or "His bundle") of the ventricular septum and thereafter to the Purkinje branches and the Purkinje muscle fibers of the right and left ventricles. This native conduction system including the His bundle, right and left branches (sometimes referred to as the right and left bundle branches) and the Purkinje fibers may be referred to as the "His-Purkinje conduction system" or "His-Purkinje system." The heart rate arising from the SA node can be referred to as the "sinus rate."

Patients with a conduction system abnormality, e.g., poor AV node conduction, poor SA node function, or other conduction abnormalities, may receive a pacemaker to restore a more normal heart rhythm and atrioventricular synchrony. Ventricular pacing may be performed to maintain the ventricular rate in a patient having atrioventricular conduction abnormalities. A single chamber ventricular pacemaker may be coupled to a transvenous ventricular lead carrying electrodes placed in the right ventricle, e.g., in the right ventricular apex. The pacemaker itself can be implanted in a subcutaneous pocket with the transvenous ventricular lead tunneled to the subcutaneous pocket. Leadless, intracardiac pacemakers have been proposed or are commercially available for implantation entirely within a heart chamber, eliminating the need for transvenous leads. An intracardiac pacemaker may provide sensing and pacing from within a chamber of the patient's heart.

For example, a leadless intracardiac pacemaker may be implanted in a ventricular chamber of a patient having AV conduction block to deliver ventricular pacing to provide ventricular rate support. Such a pacemaker may sense R-wave signals attendant to intrinsic ventricular depolarizations and deliver ventricular pacing pulses in the absence of sensed R-waves. While single chamber ventricular sensing and pacing by an intracardiac ventricular pacemaker may adequately address some patient conditions, some patients may benefit from atrial and ventricular (dual chamber) sensing for providing atrial-synchronized ventricular pacing in order to maintain a regular heart rhythm. US 2020/179708 A1 relates to a method and apparatus For establishing parameters for cardiac event detection. US 2021/236825 A1 relates to a method and apparatus for adjusting control parameters for cardiac event detection.

### SUMMARY

The techniques of this disclosure generally relate to a pacemaker configured to sense atrial event signals from a cardiac signal and deliver ventricular pacing pulses synchronized to the atrial event signals in an atrial synchronous ventricular pacing mode. The cardiac signal may be a cardiac mechanical signal representative of cardiac motion due to heart chamber contraction and relaxation. The cardiac signal can be sensed by a sensor of the pacemaker and may be sensed as an acceleration signal by an accelerometer of the pacemaker. The pacemaker may be configured to sense atrial event signals from the cardiac signal and deliver a ventricular pacing pulse upon expiration of an AV pacing interval started in response to a sensed atrial event signal to provide atrial synchronous ventricular pacing.

A ventricular pacemaker operating according to the techniques disclosed herein is configured to determine a low AV synchronous (AVS) pacing condition while operating in an atrial synchronous ventricular pacing mode. The pacemaker may determine a low AVS pacing condition based on ventricular event data. For example, the pacemaker may determine a low AVS pacing condition when the number or percentage of ventricular events that are AVS pacing pulses is less than a threshold number or threshold percentage. In response to determining a low AVS pacing condition, the pacemaker can be configured to analyze ventricular event data and/or data determined from the cardiac signal used to sense atrial event signals for determining a cause of the low AVS pacing condition. Based on the determined cause of the low AVS pacing condition, the pacemaker may select or adjust one or more operating control parameter settings for promoting an increase in the relative frequency of occurrence of ventricular events that are AVS pacing pulses.

In one example, the disclosure provides a medical device including a control circuit configured to determine a low atrioventricular synchronous pacing condition based on ventricular event data and determine a cause of the low atrioventricular synchronous pacing condition based on the ventricular event data and/or cardiac signal data. The control circuit is configured to select at least one operating control parameter setting based on the determined cause of the low atrioventricular synchronous pacing condition and operate according to the at least one selected operating control parameter setting. The medical device includes a pulse generator controlled by the control circuit to deliver ventricular pacing pulses while the control circuit is operating according to the at least one selected operating control parameter setting.

In another example, the disclosure provides a non-claimed method including determining a low atrioventricular synchronous pacing condition based on ventricular event data, determining a cause of the low atrioventricular synchronous pacing condition based on at least one of the ventricular event data and cardiac signal data. The method may include selecting at least one operating control parameter setting based on the determined cause of the low atrioventricular synchronous pacing condition. The method may include delivering ventricular pacing pulses by a pulse generator of a medical device while operating according to the at least one selected operating control parameter setting.

In another example, the disclosure provides a non-claimed non-transitory, computer-readable storage medium storing a set of instructions which, when executed by a control circuit of a medical device, cause the medical device to determine a low atrioventricular synchronous pacing condition based on ventricular event data and determine a cause of the low atrioventricular synchronous pacing condition based on at least one of the ventricular event data and cardiac signal data. The instructions may further cause the medical device to select at least one operating control parameter setting based on the determined cause of the low atrioventricular synchronous pacing condition and deliver ventricular pacing pulses by a pulse generator of the medical device while operating according to the at least one selected operating control parameter setting.

In another example, the disclosure provides a non-claimed medical device including a control circuit configured to determine a low atrioventricular synchronous pacing condition based on ventricular event data and determine a cause of the low atrioventricular synchronous pacing condition based on at least one of the ventricular event data and cardiac signal data. The medical device may include a pulse generator controlled by the control circuit to deliver ventricular pacing pulses.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a conceptual diagram illustrating a medical device system that may be used to sense cardiac signals and deliver ventricular pacing to a patient's heart.
FIG. 2 is a conceptual diagram of the pacemaker shown in FIG. 1.
FIG. 3 is a conceptual diagram of a pacemaker implanted in an alternative location within the heart for sensing cardiac signals and for delivering ventricular pacing pulses.
FIG. 4 is a conceptual diagram of an example configuration of the pacemaker of FIG. 1.
FIG. 5 is an example of a cardiac acceleration signal that may be acquired by a motion sensor of the pacemaker of FIG. 4 over a cardiac cycle.
FIG. 6 is an example of cardiac acceleration signals acquired over two different cardiac cycles.
FIG. 7 is a flow chart of a method that may be performed by the pacemaker of FIG. 4 for detecting a low AVS pacing condition, troubleshooting a cause of the low AVS pacing synchrony and selecting a corrective action based on a determined cause.
FIG. 8 is a flow chart of a method for accumulating ventricular event data and cardiac signal data that can be used for determining and troubleshooting a low AVS pacing condition according to some examples.
FIG. 9 is a flow chart of a method that may be performed by the pacemaker of FIG. 4 for determining when a slow atrial rate is a cause of a low AVS pacing condition according to some examples.
FIG. 10 is a flow chart of a method that may be performed by the pacemaker of FIG. 4 for determining when a fast atrial rate criteria is a cause of a low AVS pacing condition according to some examples.
FIG. 11A and FIG. 11B are example histogram plots of cardiac acceleration signal maximum amplitudes obtained during a time window corresponding to ventricular passive filling.
FIG. 12 is a flow chart of a method that may be performed by the pacemaker of FIG. 4 for determining when low atrial signal amplitude is a cause of a low AVS pacing condition according to some examples.
FIG. 13 is a flow chart of a method that may be performed by the pacemaker of FIG. 4 for determining an AVS pacing condition and providing a corrective action according to other examples.

### DETAILED DESCRIPTION

In general, this disclosure describes techniques for a medical device to determine a relative rate of occurrence of ventricular events that are AVS pacing pulses and select one or more operating control parameter settings used by a pacemaker for sensing atrial event signals and delivering ventricular pacing pulses to promote AVS pacing. As described herein, atrial event signals may be sensed from a cardiac signal received by processing circuitry of a ventricular pacemaker. The cardiac signal may be a cardiac mechanical signal sensed by a sensor of the pacemaker. The mechanical signal may be a pressure signal, a heart sound signal, an impedance signal, an acceleration signal, a flow signal or another mechanical signal that is responsive to atrial mechanical contraction. In other examples, the cardiac signal may be an electrical signal sensed using electrodes coupled to the pacemaker. The electrical signal may include P-waves attendant to the electrical depolarization of the atria.

In some examples, the cardiac signal is sensed by a sensor included in the pacemaker, which may be a leadless pacemaker implanted in or on a heart chamber for delivering ventricular pacing, e.g., in or on the right or left ventricle and in some examples in an atrial chamber. Atrial event signals can be sensed from a cardiac signal for use in controlling the timing of AVS pacing pulses. The atrial event signals may be sensed from a cardiac mechanical signal and correspond to motion caused by atrial mechanical contraction during atrial systole and the active filling phase of the ventricle, sometimes referred to as the "atrial kick." In this way, atrial-synchronized ventricular pacing pulses can be delivered by a pacemaker implanted in or on the ventricle in some examples, without requiring a sensor in or on the atria of the patient's heart for detecting atrial events and without requiring a lead extending from the pacemaker.

A "cardiac cycle" refers to one cycle of the heartbeat, e.g., one ventricular systolic phase followed by one ventricular diastolic phase. During an AVS pacing cardiac cycle, a ventricular pacing pulse is delivered at an AV pacing interval from the sensed atrial event signal so that the ventricular contraction follows synchronously with the atrial contraction or atrial kick. In a non-AVS pacing cardiac cycle a ventricular pacing pulse is delivered at a ventricular pacing interval from a preceding ventricular event, e.g., at a lower rate interval, a temporary rate response interval (during increased patient physical activity) or a rate smoothing interval. The atrial systolic event may occur at any time during the cardiac cycle and may not occur at all during the cardiac cycle, e.g., if the sinus rate is slower than the asynchronous ventricular paced rate.

According to the techniques disclosed herein, a ventricular pacemaker may determine a metric of AVS pacing for determining a low AVS pacing condition. A low AVS pacing condition may be determined when a percentage of AVS pacing pulses, out of all ventricular events (e.g., delivered pacing pulses and sensed R-waves) is less than a threshold percentage. The pacemaker may analyze ventricular event data and/or cardiac signal data when the low AVS pacing condition is detected. When a low AVS pacing condition is determined, the pacemaker may adjust at least one operating control parameter setting to promote an increase in the relative number of AVS pacing pulses delivered. Accordingly, the techniques disclosed herein provide improvements in a pacemaker configured to deliver ventricular pacing therapy, e.g., by increasing the incidence of AVS pacing pulses (out of all ventricular events) by adjusting operating control parameters in a patient-specific manner that reduces the time and burden required by a clinician in monitoring the pacemaker performance and programming the sensing and pacing control parameters used by the pacemaker for sensing atrial event signals and delivering ventricular pacing pulses.

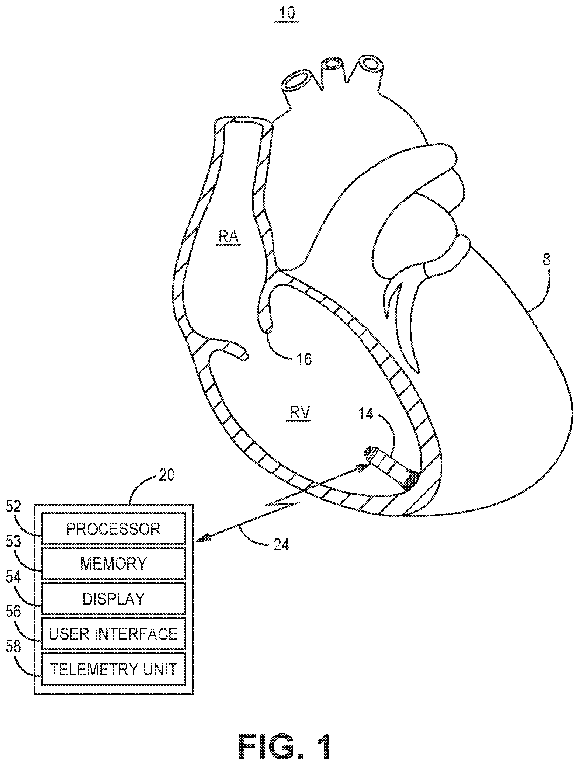

FIG. 1 is a conceptual diagram illustrating an implantable medical device (IMD) system 10 that may be used to sense cardiac signals and provide pacing therapy to a patient's heart 8. IMD system 10 includes a leadless pacemaker 14 shown implanted in a right ventricular location. Pacemaker 14 may be a transcatheter intracardiac pacemaker which is adapted for implantation wholly within a heart chamber, e.g., wholly within the right ventricle (RV) or wholly within the left ventricle (LV) of heart 8 for sensing cardiac signals and delivering ventricular pacing pulses. Pacemaker 14 may be reduced in size compared to subcutaneously implanted pacemakers and may be generally cylindrical in shape to enable transvenous implantation via a delivery catheter.

Pacemaker 14 is shown positioned in the RV, along an endocardial wall, e.g., near the RV apex though other locations are possible. The techniques disclosed herein are not limited to the pacemaker location within the RV shown in the example of FIG. 1 and other positions within or on heart 8 are possible. Pacemaker 14 may be positioned in or on the RV or LV and configured to sense atrial event signals and deliver atrial-synchronized ventricular pacing to the RV or the LV using the techniques disclosed herein. Pacemaker 14 may be positioned within the RV or LV to provide respective right ventricular or left ventricular pacing and for sensing cardiac signals by a sensor within or on the pacemaker. In some examples, pacemaker 14 may be positioned along the interventricular septum for delivering ventricular pacing pulses, which may include pacing of the left bundle branch and/or the right bundle branch of the ventricular conduction system, for example.

Pacemaker 14 is capable of producing electrical stimulation pulses, e.g., pacing pulses, delivered to heart 8 via one or more electrodes on the outer housing of the pacemaker. In the example shown, pacemaker 14 is configured to deliver RV pacing pulses and sense a raw RV cardiac electrical signal using housing based electrodes for producing an RV electrogram (EGM) signal. The cardiac electrical signals may be sensed using the housing based electrodes that are also used to deliver pacing pulses to the RV.

Pacemaker 14 is configured to control the delivery of ventricular pacing pulses to the RV in a manner that promotes synchrony between atrial activation and ventricular activation, e.g., by maintaining a target atrioventricular (AV) interval between atrial events and ventricular pacing pulses. That is, pacemaker 14 may control pacing pulse delivery to maintain a desired AV interval between atrial contractions corresponding to atrial systole and ventricular pacing pulses delivered to cause the ventricular depolarization and contraction of ventricular systole.

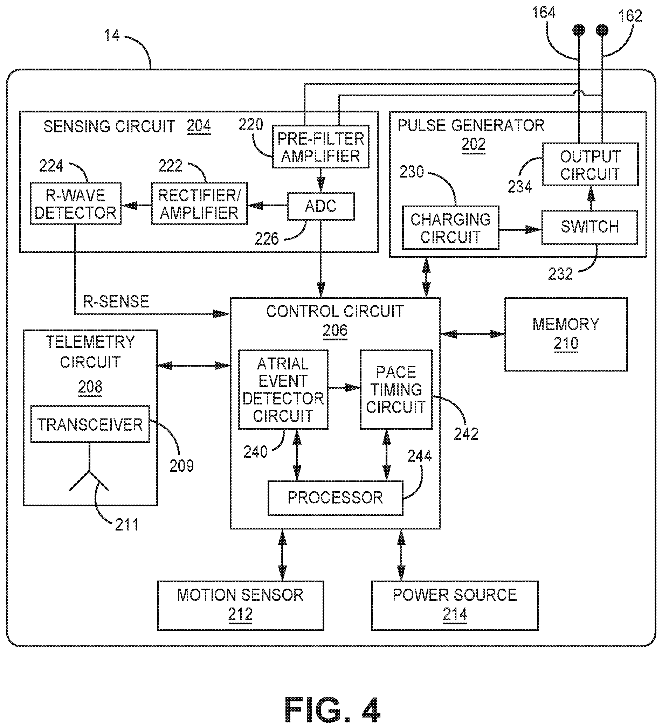

According to the techniques described herein, atrial event signals corresponding to atrial depolarization and contraction can be detected by pacemaker 14 from a cardiac signal sensed by pacemaker 14. In some examples, the sensor senses a cardiac mechanical signal. In the illustrative examples disclosed herein, the sensor is motion sensor such as an accelerometer enclosed by the housing of pacemaker 14. The motion signal produced by an accelerometer implanted within a ventricular chamber, which may be referred to as an "intraventricular motion signal," may include acceleration signals caused by ventricular and atrial mechanical events. For example, acceleration of blood flowing into the RV through the tricuspid valve 16 between the RA and RV caused by atrial systole, and referred to as the "atrial kick," may be detected by pacemaker 14 from the signal produced by an accelerometer included in pacemaker 14. Other cardiac event signals that may be present in the motion sensor signal, such as motion caused by ventricular contraction and passive ventricular filling are described below in conjunction with FIG. 4. Atrial event signals may be sensed from a variety of cardiac mechanical signals responsive to the motion of cardiac structures and/or flowing blood during a cardiac cycle including any of an impedance signal, pressure signal, flow signal, or heart sound signal.

In some examples, atrial event signals may be sensed as atrial P-waves that are attendant to atrial depolarizations from the cardiac electrical signal sensed by pacemaker 14. When pacemaker 14 is implanted in the RV, P-waves are relatively low amplitude signals in the near-field ventricular cardiac electrical signal received by pacemaker 14 (e.g., compared to the near-field R-wave) and therefore can be difficult to reliably detect from the cardiac electrical signal acquired by pacemaker 14. Atrial synchronous ventricular pacing by pacemaker 14 and any other functions that rely on atrial event signal sensing may not be reliable when based solely on a cardiac electrical signal received by pacemaker 14 when implanted in a ventricular location. According to illustrative techniques disclosed herein, pacemaker 14 may include a motion sensor and processing circuit configured to detect an atrial event signal corresponding to atrial mechanical activation or atrial systole from the signal produced by the motion sensor. Ventricular pacing pulses may be synchronized to the atrial event signal that is sensed from the motion sensor signal by setting a programmable AV pacing interval that controls the timing of the ventricular pacing pulse relative to the detected atrial systolic event, thereby promoting AV synchrony on a beat-by-beat basis.

A target AV interval may be a default value or a programmed value selected by a clinician and is the time interval from the detection of the atrial event until delivery of the ventricular pacing pulse. In some instances, the target AV interval may be started from the time the atrial event signal is sensed, e.g., based on a cardiac signal crossing an atrial event sensing threshold amplitude or starting from an identified fiducial point of the atrial event signal, e.g., a maximum peak amplitude. The target AV interval may be identified as being hemodynamically optimal for a given patient based on clinical testing or assessments of the patient or based on clinical data from a population of patients. The target AV interval may be determined to be optimal based on relative timing of electrical and mechanical events as identified from the cardiac electrical signal received by pacemaker 14 and the cardiac mechanical sensor signal received by pacemaker 14. The AV interval may be 10 to 50 ms and can be 10 to 20 ms in some examples, with no limitation intended.

Pacemaker 14 may be capable of bidirectional wireless communication with an external device 20 for programming the AV pacing interval and other pacing control parameters as well as cardiac event sensing control parameters, which may be utilized for sensing cardiac electrical events, e.g., R-waves attendant to ventricular depolarizations, and atrial event signals from the motion sensor signal. Aspects of external device 20 may generally correspond to the external programming/monitoring unit disclosed in U.S. Pat. No. 5,507,782 (Kieval, et al.). External device 20 is often referred to as a "programmer" because it is typically used by a physician, technician, nurse, clinician or other qualified user for programming operating parameters in pacemaker 14. External device 20 may be located in a clinic, hospital or other medical facility. External device 20 may alternatively be embodied as a home monitor or a handheld device that may be used in a medical facility, in the patient's home, or another location.

External device 20 may include a processor 52, memory 53, display 54, user interface 56 and telemetry unit 58. Processor 52 controls external device operations and processes data and signals received from pacemaker 14. Display unit 54 may generate a display, which may include a graphical user interface, of data and information relating to pacemaker functions to a user for reviewing pacemaker operation and programmed parameters as well as cardiac electrical signals, cardiac mechanical signals, e.g., an acceleration signal, or other physiological data that may be acquired by pacemaker 14 and transmitted to external device 20 during an interrogation session.

User interface 56 may include a mouse, touch screen, keypad or the like to enable a user to interact with external device 20 to initiate a telemetry session with pacemaker 14 for retrieving data from and/or transmitting data to pacemaker 14, including programmable parameters for controlling cardiac event sensing and therapy delivery. Telemetry unit 58 includes a transceiver and antenna configured for bidirectional communication with a telemetry circuit included in pacemaker 14 and is configured to operate in conjunction with processor 52 for sending and receiving data relating to pacemaker functions via communication link 24.

At the time of implant, during patient follow-up visits, or any time after pacemaker implantation, pacemaker 14 may perform a set-up procedure to establish parameters used in sensing atrial event signals from a cardiac signal. The patient may be standing, sitting, lying down or ambulatory during the process. The set-up procedure may include acquiring motion sensor signal data and generating distributions of motion sensor signal features for establishing atrial event sensing control parameters. Motion sensor signal data may be transmitted to external device 20 and displayed on display unit 54 of external device 20 in some examples. The atrial event sensing parameters established based on the motion sensor signal data may be set automatically or may be transmitted to external device 20 for generating a display on display unit 54 as recommended parameters, allowing a clinician to review and accept or modify the recommended parameters, e.g., using user interface 56 and interacting with a graphical user interface on display unit 54.

Telemetry unit 58 establishes a wireless communication link 24 with pacemaker 14 using a radio frequency (RF) link such as BLUETOOTH^{®}, Wi-Fi, Medical Implant Communication Service (MICS) or other communication bandwidth. In some examples, external device 20 may include a programming head that is placed proximate pacemaker 14 to establish and maintain a communication link 24, and in other examples external device 20 and pacemaker 14 may be configured to communicate using a distance telemetry algorithm and circuitry that does not require the use of a programming head and does not require user intervention to maintain a communication link.

It is contemplated that external device 20 may be in wired or wireless connection to a communications network via a telemetry circuit that includes a transceiver and antenna or via a hardwired communication line for transferring data to a centralized database or computer to allow remote management of the patient. Remote patient management systems including a centralized patient database may be configured to utilize the presently disclosed techniques to enable a clinician to review EGM, motion sensor signal, and marker channel data and authorize programming of sensing and therapy control parameters in pacemaker 14, e.g., after viewing a visual representation of EGM, motion sensor signal and marker channel data. In some examples, external device 50 is a monitoring device of a remote patient monitoring system such as a CARELINK^{™} monitor available from Medtronic, Inc., Dublin, Ireland.

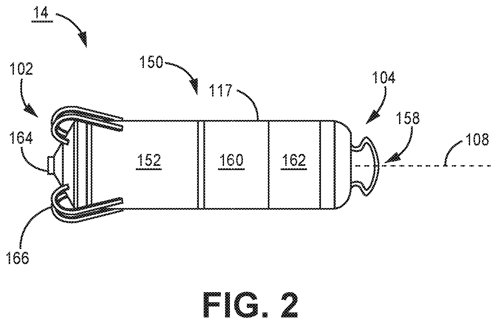

FIG. 2 is a conceptual diagram of the pacemaker 14 shown in FIG. 1 according to one example. Pacemaker 14 includes electrodes 162 and 164 spaced apart along the housing 150 of pacemaker 14 for sensing cardiac electrical signals and delivering pacing pulses. Electrode 164 is shown as a tip electrode extending from a distal end 102 of pacemaker 1. Electrode 164 is shown as a non-tissue piercing electrode that can be held in intimate contact or proximity to cardiac tissue by fixation members 166 engaging with the cardiac tissue. In other examples, tip electrode 164 may be a tissue-piercing electrode, such as a helical screw-in electrode, pointed electrode, hook electrode or the like, for penetrating the cardiac tissue at a desired pacing site. A tissue-piercing tip electrode may or may not be configured to provide fixation of pacemaker 14 at the implant site.

Electrode 162 is shown as a ring electrode along a mid-portion of housing 150, for example circumscribing the cylindrical, lateral side wall 117 of housing 150 adjacent proximal end 104. Distal end 102 is referred to as "distal" in that it is expected to be the leading end as pacemaker 14 is advanced through a delivery tool, such as a catheter, and placed against a targeted pacing site. Electrodes 162 and 164 form an anode and cathode pair for bipolar cardiac pacing and sensing.

In other examples, pacemaker 14 may include two or more ring electrodes, two tip electrodes, and/or other types of electrodes exposed along pacemaker housing 150 (on distal end 102, on proximal end 104 and/or along the lateral sidewall 117 that extends from distal end 102 to proximal end 104) for delivering electrical stimulation to heart 8 and sensing cardiac electrical signals. Electrodes 162 and 164 may be, without limitation, titanium, platinum, iridium or alloys thereof and may include a low polarizing coating, such as titanium nitride, iridium oxide, ruthenium oxide, platinum black, among others. Electrodes 162 and 164 may be positioned at locations along pacemaker 14 other than the locations shown.

Housing 150 is formed from a biocompatible material, such as a stainless steel or titanium alloy. In some examples, the housing 150 may include an insulating coating. Examples of insulating coatings include parylene, urethane, PEEK, or polyimide, among others. The entirety of the housing 150 may be insulated, but only electrodes 162 and 164 uninsulated. Electrode 164 may serve as a cathode electrode and be coupled to internal circuitry, e.g., a pacing pulse generator and cardiac electrical signal sensing circuitry, enclosed by housing 150 via an electrical feedthrough crossing housing 150. Electrode 162 may be formed as a conductive portion of housing 150 defining a ring electrode that is electrically isolated from the other portions of the housing 150 as generally shown in FIG. 2. In other examples, the entire periphery of the housing 150 may function as an electrode that is electrically isolated from tip electrode 164, instead of providing a localized ring electrode such as anode electrode 162. Electrode 162 formed along an electrically conductive portion of housing 150 serves as a return anode during pacing and sensing.

The housing 150 may include a control electronics subassembly 152, which houses the electronics for sensing cardiac signals, producing pacing pulses and controlling therapy delivery and other functions of pacemaker 14, e.g., as described below in conjunction with FIG. 4. Housing 150 may further include a battery subassembly 160, enclosing one or more chargeable or non-rechargeable batteries, which provide power to the control electronics subassembly 152, such as the various circuits and components shown in FIG. 4.

A motion sensor may be implemented as an accelerometer enclosed within housing 150 in some examples. The accelerometer provides a signal to a processor included in control electronics subassembly 152 for signal processing and analysis for sensing atrial events, e.g., for use in controlling the timing of ventricular pacing pulses during atrial synchronous ventricular pacing. The accelerometer may be a three-dimensional accelerometer. In some examples, the accelerometer may have one "longitudinal" axis that is parallel to or aligned with the longitudinal axis 108 of pacemaker 14 and two orthogonal axes that extend in radial directions relative to the longitudinal axis 108. Practice of the techniques disclosed herein, however, are not limited to a particular orientation of the accelerometer within or along housing 150. In other examples, a one-dimensional accelerometer may be used to obtain a cardiac motion signal from which atrial event signals can be sensed. In still other examples, a two dimensional accelerometer or another multi-dimensional accelerometer may be used.

Each axis of a single or multi-dimensional accelerometer may be defined by a piezoelectric element, micro-electrical mechanical system (MEMS) device or other sensor element capable of producing an electrical signal in response to changes in acceleration imparted on the sensor element, e.g., by converting the acceleration to a force or displacement that is converted to the electrical signal. In a multi-dimensional accelerometer, the sensor elements may be arranged orthogonally with each sensor element axis orthogonal relative to the other sensor element axes. Orthogonal arrangement of the elements of a multi-axis accelerometer, however, is not necessarily required.

Each sensor element may produce an acceleration signal corresponding to a vector aligned with the axis of the sensor element. A vector signal of a multi-dimensional accelerometer (also referred to as a "multi-axis" accelerometer) may be selected for use in sensing atrial event signals. In various examples, one, two or all three axis signals produced by a three-dimensional accelerometer may be selected as a vector signal for use in detecting atrial event signals, e.g., for controlling atrial synchronous ventricular pacing delivered by pacemaker 14.

Pacemaker 14 may include a set of fixation tines 166 to secure pacemaker 14 to patient tissue, e.g., by actively engaging with the ventricular endocardium and/or interacting with the ventricular trabeculae. Fixation tines 166 are configured to anchor pacemaker 14 to position electrode 164 in operative proximity to a targeted tissue for delivering therapeutic electrical stimulation pulses. Numerous types of active and/or passive fixation members may be employed for anchoring or stabilizing pacemaker 14 in an implant position. Pacemaker 14 may optionally include a delivery tool interface 158. Delivery tool interface 158 may be located at the proximal end 104 of pacemaker 14 and is configured to connect to a delivery device, such as a catheter, used to position pacemaker 14 at an implant location during an implantation procedure, for example within a heart chamber.

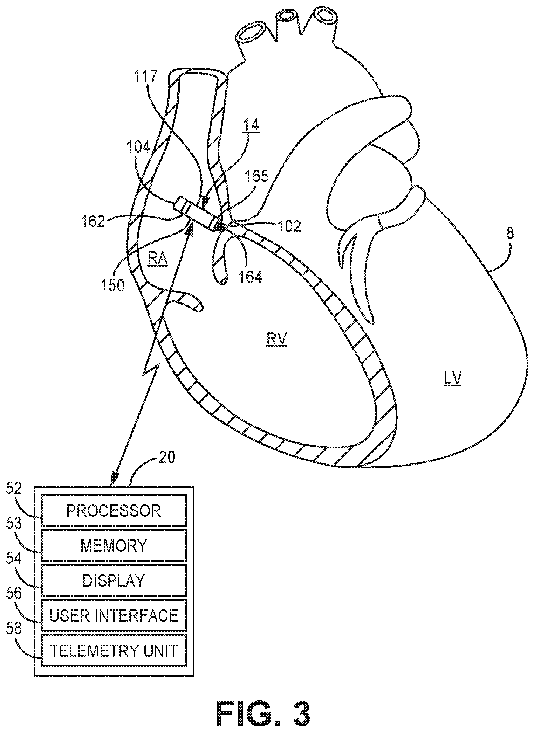

FIG. 3 is a conceptual diagram of pacemaker 14 implanted in an alternative location within the heart for sensing cardiac signals and for delivering ventricular pacing pulses. Pacemaker 14 may be positioned within the right atrium (RA) for providing ventricular pacing from an atrial implant location, which may include ventricular pacing of myocardial tissue and/or the native ventricular conduction system, which includes the His bundle, the right and left bundle branches and the Purkinje fibers and may be referred to as the "His-Purkinje system." Pacemaker 14 includes distal tip electrode 164, which in this example may be a tissue-piercing electrode, extending from the distal end 102 of the pacemaker housing 150. In FIG. 3, pacemaker 14 is shown implanted in the RA of the patient's heart to advance distal tip electrode 164 for delivering pacing pulses to or in the area of the His bundle of heart 8.

For example, the distal tip electrode 164 may be configured as a tissue piercing electrode that can be inserted into the inferior end of the interatrial septum, beneath the AV node and near the tricuspid valve annulus to position tip electrode 164 in, along or proximate to ventricular tissue, e.g., the His bundle. Distal tip electrode 164 may be a helical electrode, as shown in this example, providing fixation to anchor the pacemaker 14 at the implant position as well as deliver pacing pulses. In other examples, pacemaker 14 may include a fixation member that includes one or more tines (as shown in FIG. 1), hooks, barbs, helices or other fixation member(s) that anchor the distal end 102 of the pacemaker 14 at the implant site. Another example of a pacemaker that may be configured to operate according to the techniques disclosed herein and is configured for delivering pacing to the ventricles from an atrial implant location is generally disclosed in U.S. Patent Application No. 2019/0083800 A1 (Yang, et al.).

A portion of the distal tip electrode 164 may be electrically insulated such that only the most distal end of tip electrode 164, furthest from housing distal end 102, is exposed to provide targeted pacing at a ventricular tissue site that may include a portion of the His bundle. One or more housing-based electrodes 162 and 165 may be carried on the surface of the housing 150 of pacemaker 14. Electrodes 162 and 165 are shown as ring electrodes circumscribing the lateral sidewall 117 of pacemaker housing 150. Lateral sidewall 117 extends from distal end 102 to proximal end 104. In other examples, a return anode electrode used in sensing and pacing may be positioned on housing proximal end 104. Pacing of the ventricles may be achieved using the distal tip electrode 102 as the cathode electrode and either of the housing-based electrodes 162 or 165 as the return anode.

Cardiac electrical signals produced by heart 8 may be sensed by pacemaker 14 using a sensing electrode pair selected from electrodes 162, 164 and 165. For example, a cardiac electrical signal may be sensed using distal tip electrode 164 and distal housing-based electrode 165 or distal tip electrode 164 and proximal housing-based electrode 162. A second cardiac electrical signal may be sensed using electrodes 165 and 162. In other examples, a single cardiac electrical signal may be sensed using a single electrode pair selected from electrodes 162, 164 and 165.

Atrial synchronous or asynchronous ventricular pacing pulses may be delivered via electrodes 164 and 165, e.g., to capture at least a portion of the ventricular myocardium and/or His-Purkinje system. Pacemaker 14 may include an accelerometer or other mechanical signal sensor that can be used to sense atrial event signals for delivering atrial synchronized ventricular pacing pulses. When implanted in the RA, however, atrial P-waves attendant to atrial depolarization may be sensed from a cardiac electrical signal sensed using housing-based electrodes, e.g., electrodes 165 and 162. Pacemaker 14 may be configured to deliver ventricular pacing pulses at an AV pacing interval following sensed P-waves in some examples and/or following atrial event signals sensed from a cardiac motion signal sensed by pacemaker 14 when P-waves are not being sensed or when P-wave sensing is determined to be unreliable, e.g., in the presence of electrical noise or other oversensing. While sensing of atrial event signals from within the RA on a beat-to-beat basis may be less challenging than sensing of atrial event signals from within the RV, the techniques disclosed herein may be implemented in a pacemaker implanted in a RA location for delivering atrial synchronous ventricular pacing pulses to enable the pacemaker to detect a low AVS pacing condition and perform a corrective action.

FIG. 4 is a conceptual diagram of pacemaker 14 shown in FIGs. 1-3 according to some examples. Pacemaker 14 includes a pulse generator 202, a cardiac electrical signal sensing circuit 204, a control circuit 206, memory 210, telemetry circuit 208, motion sensor 212 and a power source 214. The various circuits represented in FIG. 4 may be combined on one or more integrated circuit boards which include an application specific integrated circuit (ASIC), an electronic circuit, a processor (shared, dedicated, or group) and memory that execute one or more software or firmware programs, a combinational logic circuit, state machine or other suitable components that provide the described functionality.

Motion sensor 212 includes an accelerometer in the examples described herein. Examples of accelerometers that may be implemented in motion sensor 212 include piezoelectric sensors and MEMS devices. Motion sensor 212 may be implemented as other types of accelerometer sensors and is not necessarily limited to being an accelerometer. Other sensors may be utilized successfully in pacemaker 14 for sensing a cardiac signal representative of cardiac motion arising from the mechanical contraction and relaxation of the heart chambers for use in sensing atrial event signals and determining a cause of a low AVS pacing condition according to the techniques described herein. Such sensors may include blood flow sensors, impedance sensors (e.g., an impedance signal correlated to heart chamber volume changes), pressure sensors and heart sound sensors. As used herein, therefore, the term "motion sensor" can refer to any sensor that produces a signal responsive to the mechanical motion of the heart chambers. For the sake of illustration, in examples presented herein, motion sensor 212 can be an accelerometer and the motion sensor signal passed from motion sensor 212 to control circuit 206 can be an acceleration signal.

Motion sensor 212 may include a multi-axis sensor, e.g., a two-dimensional or three-dimensional sensor, with each axis providing an axis signal that may be analyzed individually or in combination for detecting cardiac mechanical events. Motion sensor 212 produces an electrical signal correlated to motion or vibration of sensor 212 (and pacemaker 14), e.g., when subjected to flowing blood and cardiac motion. The motion sensor 212 may include one or more filter, amplifier, rectifier, analog-to-digital converter (ADC) and/or other components for producing a motion signal that is passed to control circuit 206. For example, each vector signal produced by each individual axis of a multi-axis accelerometer may be filtered by a high pass filter, e.g., a 3 to 15 Hz high pass filter or a 10 Hz high pass filter. The filtered signal may be digitized by an ADC and rectified for use by atrial event detector circuit 240 of control circuit 206 for detecting atrial event signals. The high pass filter may be lowered (e.g., to 5 Hz) if needed to detect atrial signals that have lower frequency content. In some examples, high pass filtering is performed with no low pass filtering. In other examples, each accelerometer axis signal is filtered by a low pass filter, e.g., a 25 Hz, 30 Hz or 40 Hz low pass filter, with or without high pass filtering.

One example of an accelerometer for use in implantable medical devices that may be implemented in conjunction with the techniques disclosed herein is generally disclosed in U.S. Pat. No. 5,885,471 (Ruben, et al.). An implantable medical device arrangement including a piezoelectric accelerometer for detecting patient motion is disclosed, for example, in U.S. Pat. No. 4,485,813 (Anderson, et al.) and U.S. Pat. No. 5,052,388 (Sivula, et al.). Examples of three-dimensional accelerometers that may be implemented in pacemaker 14 and used for detecting cardiac mechanical events using the presently disclosed techniques are generally described in U.S. Pat. No. 5,593,431 (Sheldon) and U.S. Pat. No. 6,044,297 (Sheldon). Other accelerometer designs may be used for producing an electrical signal that is correlated to acceleration forces imparted on pacemaker 14 due to ventricular and atrial events.

Sensing circuit 204 is configured to receive a cardiac electrical signal via electrodes 162 and 164 by a pre-filter and amplifier circuit 220. Pre-filter and amplifier circuit 220 may include a high pass filter to remove DC offset, e.g., a 2.5 to 5 Hz high pass filter, or a wideband filter having a passband of 2.5 Hz to 100 Hz to remove DC offset and high frequency noise. Pre-filter and amplifier circuit 220 may further include an amplifier to amplify the "raw" cardiac electrical signal passed to analog-to-digital converter (ADC) 226. ADC 226 may pass a multi-bit, digital cardiac electrogram (EGM) signal to control circuit 206 for use in identifying ventricular electrical events (e.g., R-waves or T-waves) and/or atrial electrical events, e.g., P-waves. Identification of cardiac electrical events, e.g., R-waves, may be used by control circuit 206 in algorithms for establishing atrial sensing control parameters and for detecting atrial event signals from the motion sensor signal. The digital signal from ADC 226 may be passed to rectifier and amplifier circuit 222, which may include a rectifier, bandpass filter, and amplifier for passing a cardiac signal to R-wave detector 224.

R-wave detector 224 may include a sense amplifier or other detection circuitry that compares the incoming rectified, cardiac electrical signal to an R-wave sensing threshold, which may be an auto-adjusting threshold. When the incoming signal crosses the R-wave sensing threshold, the R-wave detector 224 produces a ventricular sensed event signal (Vsense) that is passed to control circuit 206. In other examples, R-wave detector 224 may receive the digital output of ADC 226 for detecting R-waves by a comparator, morphological signal analysis of the digital EGM signal or other R-wave detection techniques.

Processor 244 may provide sensing control signals to sensing circuit 204, e.g., the R-wave sensing sensitivity and various blanking and refractory intervals applied to the cardiac electrical signal for controlling R-wave sensing. Ventricular sensed event signals (Vsense signals) passed from R-wave detector 224 to control circuit 206 may be used for inhibiting and/or scheduling ventricular pacing pulses by pace timing circuit 242, determining ventricular cycle lengths, and for setting a post-ventricular atrial blanking period (PVAB), a refractory period and/or various sensing windows used by atrial event detector circuit 240 for sensing atrial event signals from an acceleration signal received from motion sensor 212.

Control circuit 206 includes an atrial event detector circuit 240, pace timing circuit 242, and processor 244. Control circuit 206 may receive ventricular sensed event signals and/or digital cardiac electrical signals from sensing circuit 204 for use in detecting and confirming cardiac events and controlling ventricular pacing. For example, ventricular sensed event signals may be passed to pace timing circuit 242 for inhibiting scheduled ventricular pacing pulses or scheduling ventricular pacing pulses when pacemaker 14 is operating in a non-atrial tracking (asynchronous) ventricular pacing mode.

Atrial event detector circuit 240 is configured to detect atrial event signals from a motion signal received from motion sensor 212. Atrial event detector circuit 240 may start a PVAB and a post-ventricular atrial refractory period in response to a ventricular electrical event, e.g., a ventricular sensed event signal from sensing circuit 204 or delivery of a ventricular pacing pulse by pulse generator 202. The blanking period may correspond to a time period after the ventricular electrical event during which ventricular systolic events, e.g., corresponding to ventricular contraction are expected to occur. When ventricular pacing is properly synchronized to atrial events, an atrial event (e.g., atrial depolarization and contraction and corresponding atrial kick) is not expected to occur during the blanking period, corresponding to ventricular systole. The PVAB may be applied to define a time period following a ventricular electrical event during which an atrial event signal is not sensed by atrial event detector circuit 240. The motion sensor signal may or may not be received or processed by control circuit 206 during the atrial blanking period. For example, during atrial event sensing for controlling atrial synchronous ventricular pacing, the motion sensor may be at least partially powered off to conserve power source 214 unless an active telemetry session is in progress such that the motion sensor signal may be received by control circuit 206, including during the blanking period, for transmission to external device 20 during a telemetry session. In other examples, the motion signal may be received and analyzed during the atrial blanking period.

Atrial event detector circuit 240 determines if the motion sensor signal satisfies atrial event detection criteria outside of the atrial blanking period. The motion sensor signal during the atrial blanking period may be monitored by atrial event detector circuit 240 for the purposes of detecting ventricular mechanical events, which may be used for confirming or validating atrial systolic event detection in some examples. As described below, a peak amplitude of the motion signal may be used for analyzing the motion signal when a low AVS pacing condition is determined by control circuit 206. Atrial event detector circuit 240 may be configured to detect one or more ventricular mechanical events, or at least detect motion signal maximum amplitudes and/or threshold crossings, during the PVAB and/or refractory period in some examples. The timing and detection of the ventricular mechanical events may be used to update the atrial blanking period or other atrial sensing control parameters and/or may be used to confirm detection of the atrial event occurring subsequent to expected ventricular mechanical events. The maximum amplitude of the motion signal during the PVAB may be determined for identifying a low amplitude condition of the motion signal that may be associated with atrial event signal undersensing and a low AVS pacing condition. The amplitude of the motion signal during a latest portion of the PVAB period may be determined for use in shortening (or lengthening) the PVAB period in some examples.

Atrial event detector circuit 240 may set a time window corresponding to the passive ventricular filling phase of a cardiac cycle based on the timing of a preceding ventricular electrical event, either an R-wave sensed event signal received from sensing circuit 204 or a ventricular pacing pulse delivered by pulse generator 202. A motion sensor signal crossing of an atrial event sensing threshold during or after the passive ventricular filling time window may be detected as the atrial event signal by atrial event detector circuit 240. As described below, two different atrial event sensing threshold amplitudes may be applied, a first higher sensing threshold amplitude during the passive filling window and a second lower sensing threshold amplitude after the passive filling window.

Atrial event detector circuit 240 passes an atrial event detection signal to processor 244 and/or pace timing circuit 242 in response to detecting an atrial event signal. Pace timing circuit 242 (or processor 244) may additionally receive ventricular sensed event signals from R-wave detector 224 for use in controlling the timing of pacing pulses delivered by pulse generator 202. Processor 244 may include one or more clocks for generating clock signals that are used by pace timing circuit 242 to time out an AV pacing interval, sometimes referred to as an "AV delay," that is started upon receipt of an atrial event detection signal from atrial event detector circuit 240 when control circuit 206 is operating in an atrial synchronous ventricular pacing mode, e.g., a VDD pacing mode.

Pace timing circuit 242 may include one or more pacing escape interval timers or counters that are used to time out the AV pacing interval and other pacing escape intervals, e.g., a lower rate interval, a rate smoothing interval or a rate response pacing interval. The AV pacing interval may be a programmable interval stored in memory 210 and retrieved by processor 244 for use in setting the AV pacing interval used by pace timing circuit 242. A ventricular pacing pulse delivered upon expiration of an AV pacing interval can be referred to as an "AVS pacing pulse." Pace timing circuit 242 may include a lower rate interval timer for controlling a minimum ventricular pacing rate. For example, if an atrial event signal is not detected from the motion sensor signal triggering a ventricular pacing pulse at the programmed AV pacing interval, a ventricular pacing pulse may be delivered by pulse generator 202 upon expiration of the lower rate interval (corresponding to the programmed pacing lower rate) to prevent ventricular asystole and maintain a minimum ventricular rate. Ventricular pacing pulses delivered asynchronously to atrial events, e.g., upon expiration of a lower rate interval, a rate smoothing interval, or a rate response interval instead of an AV pacing interval, can be referred to as "non-AVS pacing pulses."

At times, control circuit 206 may control pulse generator 202 in a non-atrial tracking ventricular pacing mode (also referred to as an "asynchronous ventricular pacing mode"), which can be denoted as a VDI(R) or VVI(R) pacing mode. Ventricular pacing pulses can be delivered by pulse generator 202 in the absence of a sensed R-wave upon expiration of a ventricular lower rate interval, which may be set to a temporary rate response interval during increased patient physical activity or to a rate smoothing interval to avoid abrupt changes in pacing rate. A scheduled ventricular pacing pulse is inhibited in response to a ventricular sensed event signal from sensing circuit 204. Increased patient activity may be determined by control circuit 206 based on the motion signal received from motion sensor 212 because an acceleration signal from motion sensor 212, for example, will include acceleration signals due to patient physical activity.

Control circuit 206 may establish control parameters used for sensing atrial event signals based on analysis of the motion signal sensed during a non-atrial tracking pacing mode, e.g., as generally disclosed in U.S. Patent Application No. 16/703,047 (Splett, et al.) and U.S. Patent Application No. 16/703,320 (Splett, et al.). The non-atrial tracking ventricular pacing mode may include a VVI pacing mode, during which atrial event signals are not sensed but the motion signal may be analyzed. The VVI pacing mode may be followed by a VDI pacing mode in which atrial event signals are sensed for use in further adjusting the atrial event sensing control parameters but not used for triggering a ventricular pacing pulse. Ventricular pacing pulses are delivered or inhibited during the VVI or VDI pacing modes based on whether a ventricular sensed event signal is received from sensing circuit 204.

Dual chamber sensing may be performed during the non-atrial tracking ventricular pacing mode by sensing ventricular electrical events by sensing circuit 204 and sensing atrial event signals from the motion signal received by atrial event detector circuit 240 from motion sensor 212. Atrial event sensing parameters that can be established by control circuit 206 during a VVI and/or VDI pacing mode may include an atrial event sensing vector of the motion sensor producing a signal from which the atrial event signal is detected, the ending time of a passive ventricular filling window, and the atrial event sensing threshold amplitude values applied during and after the passive ventricular filling window. After being initially set to starting values, the sensing control parameters may be adjusted by control circuit 206 based on analysis of the motion signal during an atrial synchronous ventricular pacing mode, e.g., a VDD pacing mode, using the techniques generally disclosed in the U.S. Patent Application No. 17/159,596 (Sheldon, et al.) and U.S. Patent Application No. 17,159,635 (Sheldon, et al.).

Pulse generator 202 generates electrical pacing pulses that are delivered to the ventricles of the patient's heart via cathode electrode 164 and return anode electrode 162. In addition to providing control signals to pace timing circuit 242 and pulse generator 202 for controlling the timing of ventricular pacing pulses, processor 244 may retrieve programmable pacing control parameters, such as pacing pulse amplitude and pacing pulse width, which are passed to pulse generator 202 for controlling pacing pulse delivery. Pulse generator 202 may include charging circuit 230, switching circuit 232 and an output circuit 234.

Charging circuit 230 may include a holding capacitor that may be charged to a pacing pulse amplitude by a multiple of the battery voltage signal of power source 214 under the control of a voltage regulator. The pacing pulse amplitude may be set based on a control signal from control circuit 206. Switching circuit 232 may control when the holding capacitor of charging circuit 230 is coupled to the output circuit 234 for delivering the pacing pulse. For example, switching circuit 232 may include a switch that is activated by a timing signal received from pace timing circuit 242 upon expiration of an AV pacing interval (or ventricular lower rate interval) and kept closed for a programmed pacing pulse width to enable discharging of the holding capacitor of charging circuit 230. The holding capacitor, previously charged to the pacing pulse voltage amplitude, is discharged across electrodes 162 and 164 through the output capacitor of output circuit 234 for the programmed pacing pulse duration. Examples of pacing circuitry generally disclosed in U.S. Pat. No. 5,507,782 (Kieval, et al.) U.S. Pat. No. 8,532,785 (Crutchfield, et al.),
may be implemented in pacemaker 14 for charging a pacing capacitor to a predetermined pacing pulse amplitude under the control of control circuit 206 and delivering a pacing pulse.

Memory 210 may include computer-readable instructions that, when executed by control circuit 206, cause control circuit 206 to perform various functions attributed throughout this disclosure to pacemaker 14. The computer-readable instructions may be encoded within memory 210. Memory 210 may include any non-transitory, computer-readable storage media including any volatile, non-volatile, magnetic, optical, or electrical media, such as a random access memory (RAM), read-only memory (ROM), non-volatile RAM (NVRAM), electrically-erasable programmable ROM (EEPROM), flash memory, or other digital media with the sole exception being a transitory propagating signal. Memory 210 may store timing intervals and other data used by control circuit 206 to control the delivery of pacing pulses by pulse generator 202, e.g., by detecting an atrial event by atrial event detector circuit 240 from the motion sensor signal and setting an AV pacing interval timer included in pace timing circuit 242.

Memory 210 may store ventricular event data as a history of ventricular events, which may include a history of ventricular pacing pulse delivery and a history of ventricular sensed event signals. For example, control circuit 206 and memory 210 may track or log the number of ventricular pacing pulses delivered during a monitoring time period, which may be the time since pacemaker implant, the most recent month, most recent week, most recent day, most recent hour, or any other monitoring time period or multiple monitoring time periods of different lengths and/or different starting and/or ending times. The number of ventricular pacing pulses tracked and stored in memory 210 for a monitoring time period may include the total number of all ventricular pacing pulses, the total number of AVS pacing pulses delivered at an AV interval following a sensed atrial event signal, and the total number of non-AVS pacing pulses delivered at a ventricular pacing interval from a preceding ventricular pacing pulse or ventricular sensed event signal. The number of ventricular sensed event signals received from sensing circuit 204 may be stored in memory 210 for each of the monitoring periods that ventricular pacing pulse histories are stored. As described below, control circuit 206 may analyze the ventricular event data for detecting a low AVS pacing condition.

Control circuit 206 may determine cardiac signal data, such as motion signal features, for use in troubleshooting a low incidence of AVS pacing pulses. For example, control circuit 206 may determine one or more peak amplitudes of the motion signal during a ventricular cycle. A maximum amplitude of the rectified motion signal during the PVAB and/or after the PVAB may be determined and stored for each one of multiple ventricular cycles. In some examples, a maximum amplitude during the passive ventricular filling window and/or after the passive ventricular filling window may be determined and stored in memory 210 for each of multiple ventricular cycles. Maximum amplitudes of the motion signal corresponding to sensed atrial event signals and/or during PVABs may be stored in memory 210 as cardiac signal data, e.g., in histogram bins allocated in memory 210. In various examples, one or more maximum peak amplitudes of the motion signal may be stored in memory 210 for each ventricular cycle during the same monitoring period or any portion of the monitoring period over which ventricular event counts are stored. Each maximum peak amplitude may be stored with a corresponding timing indicator. The timing indicator may designate the maximum peak amplitude as being within a specified timing window (such as an atrial blanking period, during the passive ventricular filling window or after the passive ventricular filling window). Additionally or alternatively, the timing indicator may be a determined sample point number or a time interval from the most recent preceding ventricular event.

Power source 214, which may be enclosed in battery subassembly 160 (FIG. 2), provides power to each of the other circuits and components of pacemaker 14 as required. Power source 214 may include one or more energy storage devices, such as one or more rechargeable or non-rechargeable batteries. The connections between power source 214 and other pacemaker circuits and components are not shown in FIG. 4 for the sake of clarity but are to be understood from the general block diagram of FIG. 4. For example, power source 214 may provide power as needed to charging and switching circuitry included in pulse generator 202, amplifiers, ADC 226 and other components of sensing circuit 204, telemetry circuit 208, memory 210, and motion sensor 212.

Telemetry circuit 208 includes a transceiver 209 and antenna 211 for wirelessly transmitting and receiving data, e.g., via a radio frequency (RF) communication link. Telemetry circuit 208 may be capable of bi-directional communication with external device 20 (Fig. 1) as described above. Cardiac signals and/or data derived therefrom may be transmitted by telemetry circuit 208 to external device 20. Programmable control parameters and algorithms for performing atrial event signal sensing and ventricular pacing control may be received by telemetry circuit 208 and stored in memory 210 for access by control circuit 206.

The functions attributed to pacemaker 14 herein may be embodied as one or more processors, controllers, hardware, firmware, software, or any combination thereof. Depiction of different features as specific circuitry is intended to highlight different functional aspects and does not necessarily imply that such functions must be realized by separate hardware, firmware or software components or by any particular circuit architecture. Rather, functionality associated with one or more circuits described herein may be performed by separate hardware, firmware or software components, or integrated within common hardware, firmware or software components. For example, atrial event signal sensing from the motion sensor signal and ventricular pacing control operations performed by pacemaker 14 may be implemented in control circuit 206 executing instructions stored in memory 210 and relying on input from sensing circuit 204 and motion sensor 212. Providing software, hardware, and/or firmware to accomplish the described functionality in the context of any modern pacemaker, given the disclosure herein, is within the abilities of one of skill in the art.

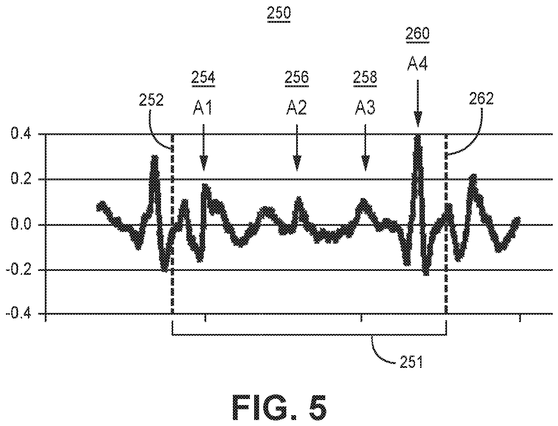

FIG. 5 is an example of an acceleration signal 250 that may be acquired by motion sensor 212 over a cardiac cycle. Vertical dashed lines 252 and 262 denote the timing of two consecutive ventricular events (an intrinsic ventricular depolarization, e.g., a sensed R-wave, or a delivered ventricular pacing pulse), marking the respective beginning and end of the cardiac cycle 251 that includes one ventricular systolic phase and one ventricular diastolic phase. The motion signal includes an A1 event 254, an A2 event 256, an A3 event 258 and an A4 event 260. The A1 event 254 is an acceleration signal that occurs during ventricular contraction and marks the approximate onset of ventricular mechanical systole. The A1 event corresponds to ventricular contraction and the ejection of blood from the ventricles. The A2 event 256 is an acceleration signal that may occur with closure of the aortic and pulmonic valves, marking the approximate offset or end of ventricular mechanical systole. The A2 event may also mark the beginning of the isovolumic relaxation phase of the ventricles that occurs with aortic and pulmonic valve closure. The A3 event 258 is an acceleration signal that occurs during passive ventricular filling and marks ventricular mechanical diastole. The A3 event signal can be referred to as the "ventricular passive filling event signal."

The A4 event 260 is an acceleration signal that occurs during atrial contraction and active ventricular filling and marks atrial mechanical systole. The A4 event 260 is also referred to herein as the "A4 signal" and is the "atrial event" or "atrial event signal" that is detected from motion sensor signal 250 by control circuit 206. For example, atrial event detector circuit 240 may detect the A4 event signal 260 in response to an atrial event sensing threshold crossing by the motion signal 250. Processor 244 may control pace timing circuit 242 (shown in FIG. 4) to trigger a ventricular pacing pulse by starting an AV pacing interval in response to atrial event detector circuit 240 detecting the A4 event 260 during an atrial synchronous ventricular pacing mode. During the atrial synchronous ventricular pacing mode, control circuit 206 may store a history of sensed A4 event signals and, in some examples, maximum amplitudes of the motion signal 250 as described in greater detail below. During a non-atrial tracking ventricular pacing mode, control circuit 206 may determine features of the motion signal 250 during and/or after an atrial blanking period.

Distributions of the features of the motion signal may be used in establishing operating control parameters used for sensing atrial event signals, e.g., as generally described in the above-referenced U.S. Patent Application No. 16/703,047 (Splett, et al.) and in U.S. Patent Application No. 16/703,320 (Splett, et al.). Furthermore, according to techniques disclosed herein, control circuit 206 may determine one or more maximum amplitudes of the motion signal during and/or outside an atrial blanking period for use in troubleshooting a detected low AVS pacing condition and selecting an operating control parameter setting that is used by control circuit 206 for controlling ventricular pacing, which may include atrial event sensing control parameters, pacing mode, pacing rate or other operating control parameters according to any of the examples presented herein.

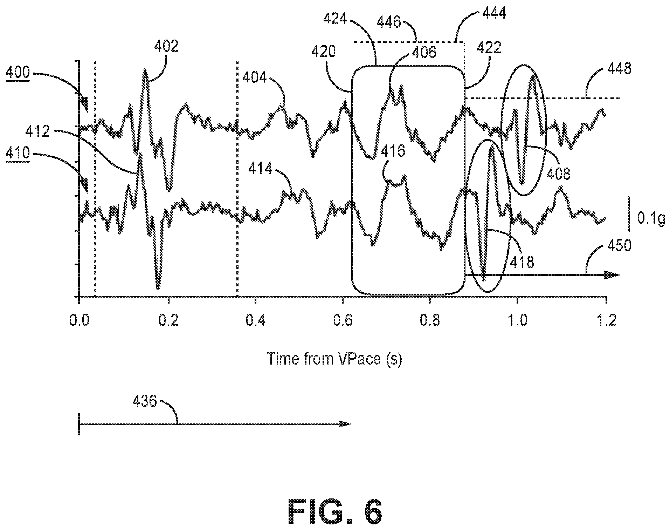

FIG. 6 is an example of motion sensor signals 400 and 410 acquired over two different cardiac cycles. A ventricular pacing pulse is delivered at time 0.0 seconds for both cardiac cycles. The top sensor signal 400 is received over one cardiac cycle, and the bottom sensor signal 410 is received over a different cardiac cycle. Each cardiac cycle includes one phase of ventricular systole followed by one phase of ventricular diastole. The two signals 400 and 410 are aligned in time at 0.0 seconds, the time of the ventricular pacing pulse delivery. While motion signals 400 and 410 and motion signal 250 of FIG. 5 are shown as raw accelerometer signals, it is recognized that control circuit 206 may receive a digitized filtered, amplified and rectified signal from motion sensor 212 for processing and analysis as described in conjunction with the flow charts and histogram distributions presented in the accompanying drawings. In other examples, control circuit 206 may receive an unrectified signal from motion sensor 212 and apply any of digital filtering, amplification and/or rectification to the raw signal.

The A1 events 402 and 412 of the respective motion sensor signals 400 and 410, which occur during ventricular contraction, are observed to be well-aligned in time following the ventricular pacing pulse at time 0.0 seconds. Similarly, the A2 events 404 and 414 (which may mark the end of ventricular systole and the isovolumic ventricular relaxation phase) and the A3 events 406 and 416 (occurring during passive ventricular filling) are well-aligned in time. Because the A1, A2 and A3 events are ventricular event signals (e.g., occurring during ventricular contraction, at the end of ventricular systole and during passive ventricular filling, respectively) these event signals are expected to occur at relatively consistent time intervals following a ventricular electrical event, the ventricular pacing pulse in this example, and relative to each other. The time relationship of the A1, A2 and A3 events may be different following a ventricular pacing pulse compared to following a sensed intrinsic R-wave and may shorten when the heart rate increases. During a stable paced or intrinsic ventricular rhythm, however, the relative timing of ventricular A1, A2 and A3 events to each other and the immediately preceding ventricular electrical event is expected to be consistent from beat-to-beat.

The A4 events 408 and 418 of the first and second motion sensor signals 400 and 410, respectively, are not aligned in time. The A4 event occurs during atrial systole and as such the time interval of the A4 event following the immediately preceding ventricular electrical event (sensed R-wave or ventricular pacing pulse) and the preceding A1 through A3 events may vary between cardiac cycles. The timing of A4 events relative to ventricular events may vary as the atrial rate changes. The timing of A4 events relative to ventricular events may be highly variable during non-atrial tracking ventricular pacing modes.

The consistency of the timing of the A1 through A3 events relative to each other and the immediately preceding ventricular electrical event may be used for determining a PVAB 436 and increasing confidence in reliably detecting A4 events 408 and 418 after the PVAB 436 during a given cardiac cycle. Control circuit 206 does not detect atrial event signals during the atrial blanking period 436, which may extend from the ventricular electrical event (at time 0.0) through an estimated onset of ventricular diastole so that the atrial blanking period 436 may include both the A1 and A2 events. In this way an A1 or A2 event is not falsely sensed as the A4 event.

A passive ventricular filling window 424, also referred to herein as the "A3 window" may be set by control circuit 206 to have a starting time 420 corresponding to the end of the PVAB 436 and an ending time 422 that is expected to be later than the A3 event. The A3 window ending time 422 may also be considered a starting time of an A4 sensing window 450, though A4 signals may be sensed during the A3 window in some instances as described below.

A4 events 408 and 418 may be sensed based on a multi-level A4 sensing threshold 444. As seen by the lower motion sensor signal 410, the A4 event 418 may occur relatively early in the A4 window 450 due to changes in atrial rate. In some instances, as the atrial rate increases, the A4 event 418 may occur within the A3 window 424. When this occurs, the A3 event 416 and the A4 event 418 may fuse as passive and active ventricular filling occur together. The fused A3/A4 event may have a high amplitude, even greater than the amplitude of either the A3 event 416 or the A4 event 418 when they occur separately. As such, in some examples a first, higher A4 sensing threshold amplitude 446 may be established and applied to the motion signal during the A3 window 424 for detecting an early A4 signal that is fused with the A3 signal. A second, lower A4 sensing threshold amplitude 448 may be established and applied to the motion signal 410 for detecting relatively later A4 signals, after the ending time 422 of the A3 window 424, during the A4 window 450.

The A4 window 450 extends from the ending time 422 of the A3 window 424 until an A4 event is detected or until the next ventricular electrical event, sensed or paced, whichever comes first. The earliest crossing of the A4 sensing threshold 444 by the motion sensor signal after the starting time 420 of the A3 window (or after the expiration of the atrial blanking period 436) may be sensed as the A4 event signal. Techniques for establishing and adjusting a first, higher A4 sensing threshold amplitude 446 applied to the motion signal during the A3 window 424 and a second, lower A4 sensing threshold amplitude 448 applied to the motion signal after the ending time 422 of the A3 window 424 are described in the above-incorporated U.S. Patent Application No. 16/703,047 (Splett, et al.), U.S. Patent Application No. 16/703,320 (Splett, et al.), U.S. Patent Application No. 17/159,596 (Sheldon, et al.) and U.S. Patent Application No. 17,159,635 (Sheldon, et al.).

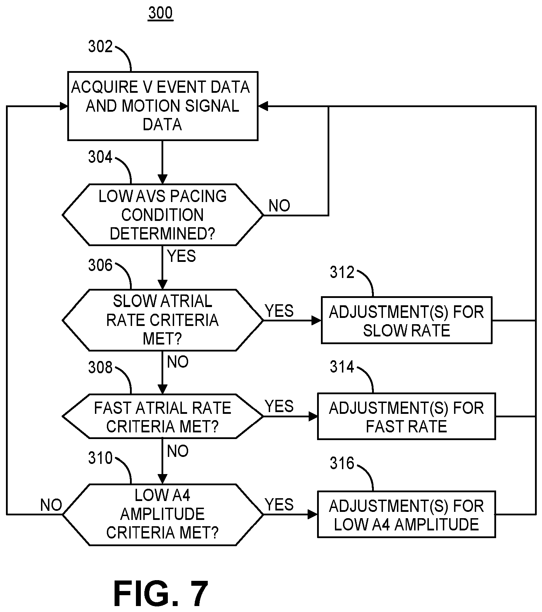

FIG. 7 is a flow chart 300 of a method that may be performed by pacemaker 14 for detecting a low AVS pacing condition and troubleshooting a cause of the low AVS pacing synchrony. The process of flow chart 300 is described as being performed by control circuit 206 of pacemaker 14 for the sake of convenience. It is to be understood, however, that in other examples another processor, alone or in combination with control circuit 206, may perform any part of the technique of FIG. 7 and other flow charts and diagrams presented herein. For example, external device processor 52 (shown in FIG. 1) or processing circuity and associated memory of another computing device alone or in combination with external device processor 52, may perform any part of the techniques disclosed herein. Processing circuitry of another device may receive ventricular event data and cardiac signal data transmitted from pacemaker 14 as needed for performing the techniques disclosed herein for detecting and troubleshooting a low AVS pacing condition.

At block 302, control circuit 206 may acquire and store ventricular event data, such as counts of ventricular pacing pulses and sensed R-waves, in memory 210. Control circuit 206 may additionally determine and store motion signal data, which may include atrial event sensing history and/or motion signal amplitude-related data. The ventricular event data is used by control circuit 206 to detect a low AVS pacing condition at block 304, as described below. The motion signal data can be used by control circuit 206, sometimes in combination with ventricular event data, to troubleshoot the low AVS pacing condition when detected. Control circuit 206 may be configured to perform a logical, systematic analysis of the data acquired at block 302 to determine a likely cause of a low AVS pacing condition and select a corrective action, e.g., by selecting one or more operating control parameter settings. The selected operating control parameter settings may be intended to promote an increase the incidence of AVS pacing pulses and/or reduce the incidence of non-AVS pacing pulses. In some examples, however, improvements in the low AVS pacing condition, e.g., increasing the percentage of AVS pacing pulses out of all ventricular events, may be limited. In this case, an operating control parameter setting may be selected by control circuit 206 to promote some minimum level of ventricular rate support, even if the ventricular pacing is not synchronous with atrial events.

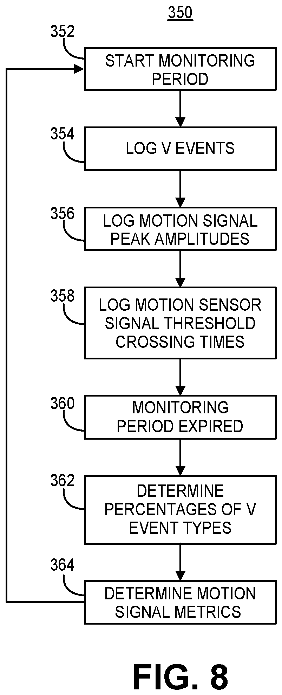

Techniques for acquiring and storing data at block 302 are described below in conjunction with FIG. 8. In general, control circuit 206 may log ventricular events so that a percentage of cardiac cycles that are AVS paced cycles and a percentage of cardiac cycles that are non-AVS paced cycles can be determined. Control circuit 206 may be configured to log in memory 210 a history of delivered ventricular pacing pulses and sensed ventricular events over a given monitoring time period, e.g., since pacemaker implant, over the most recent one week, over the most recent 24 hours and/or other time periods. For instance, control circuit 206 may log each ventricular event over a 24 hour period as either a paced ventricular event (when a ventricular pacing pulse is delivered by pulse generator 202) or a sensed ventricular event (when a ventricular sensed event signal is received from sensing circuit 204). Control circuit 206 may log each paced ventricular event as being either an AVS pacing pulse that is delivered upon expiration of an AV pacing interval started in response to a sensed atrial event signal or a non-AVS pacing pulse that is delivered upon expiration of a ventricular pacing interval started in response to a most recent preceding ventricular event.

At block 304, control circuit 206 may determine if a low AVS pacing condition is detected. Control circuit 206 may determine a low AVS pacing condition when the percentage of AVS pacing pulses out of all ventricular events logged in memory 210 over a given monitoring period is less than a threshold percentage. Low AVS pacing criteria may be met when the percentage of AVS pacing pulses (out of all delivered ventricular pacing pulses or out of all ventricular events) is less than a threshold percentage. A patient receiving pacemaker 14 is likely to be diagnosed with AV conduction block. Depending on the severity of the conduction block, all or a vast majority of ventricular events over a given monitoring time period, e.g., 24 hours or other selected monitoring period, may be ventricular pacing pulses. Accordingly, control circuit 206 may determine the percentage of AVS pacing pulses out of all delivered ventricular pacing pulses during the selected monitoring period or out of all logged ventricular events during the monitoring time period and compare the percentage of AVS pacing pulses to a low AVS pacing threshold percentage.

The low AVS pacing threshold percentage may be a fixed or programmable value that is between 50% and 95%, as examples, and may be set between 70% and 90% or to 75% in an example. When the percentage of AVS pacing pulses is less than the threshold percentage, control circuit 206 may determine a low AVS pacing condition at block 304. In other examples, control circuit 206 may detect a low AVS pacing condition in response to the percentage of AVS pacing pulses being less than a first percentage threshold and the percentage of non-AVS pacing pulses being greater than a second percentage threshold, e.g., greater than 10%, greater than 20%, or greater than 30%.

In some examples, the percentage of AVS pacing pulses is determined as a percentage of all ventricular pacing pulses that are delivered (or all ventricular events counted) while an atrial synchronous ventricular pacing mode is in effect. Ventricular pacing pulses delivered during a non-atrial tracking ventricular pacing mode, e.g., a VDI or VVI pacing mode or during rate response pacing, may be excluded when determining the percentage of AVS pacing pulses. Control circuit 206 may switch to a non-atrial tracking ventricular pacing mode to provide rate response pacing when a patient physical activity metric determined by control circuit 206 from the motion sensor signal indicates increased physical activity. Control circuit 206 may switch to a non-atrial tracking ventricular pacing mode when other mode switching criteria are met, such as the actual ventricular pacing rate exceeding a maximum atrial tracking rate limit. The total time that pacemaker 14 operates in a non-atrial tracking ventricular pacing mode may vary from day to day. Ventricular pacing history that is logged in memory during a non-atrial tracking ventricular pacing mode may be ignored for detecting a low AVS pacing condition in some examples.

In other examples, however, ventricular events logged during a non-atrial tracking ventricular pacing mode are used in determining the relative percentage of AVS pacing pulses and/or other different types of ventricular events. It is generally expected that pacemaker 14 will operate in an atrial synchronous ventricular pacing mode, e.g., a VDD pacing mode, most of the time such that any ventricular pacing pulses delivered during a non-atrial tracking pacing mode may account for an insignificant percentage of the logged ventricular events, e.g., less than 5% or less than 2% of the logged ventricular events.

Control circuit 206 may be configured to determine a low AVS pacing condition based on the ventricular event data logged in memory 210 at one or more scheduled times of day or at scheduled time intervals, which may be anywhere between one minute and one week time intervals in various examples. For example, control circuit 206 may determine if a low AVS pacing condition is detected at block 304 after every 1, 2, 4, 8, 12, 24, 48, or 72 hours, as examples, or after any other specified monitoring time period. In some examples, the process for determining a low AVS pacing condition is present is performed daily. When a low AVS pacing condition is not determined at block 304, control circuit 206 returns to block 302 to wait for the next monitoring period to elapse and reevaluates the updated ventricular event history in memory 210.

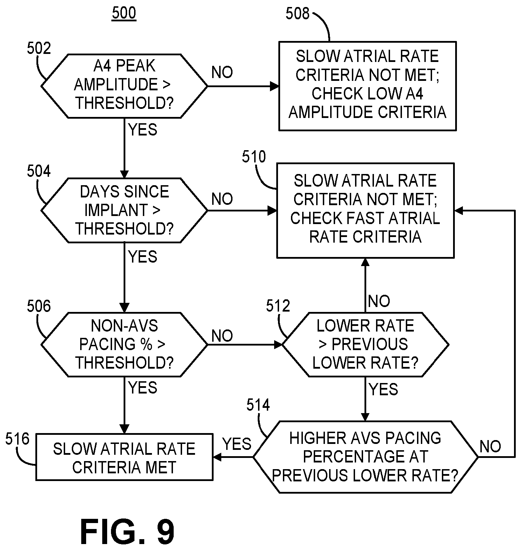

When a low AVS pacing condition is detected at block 304, control circuit 206 may advance to block 306 to troubleshoot the low AVS pacing condition for determining a likely cause of the relatively low percentage of AVS pacing pulses. Control circuit 206 may be configured to determine the cause of the low AVS pacing condition as being a slow atrial rate (block 306), a fast atrial rate (block 308) or a low atrial event signal amplitude (block 310). Each of these conditions may cause atrial event detector circuit 240 to miss or undersense some atrial event signals. When an atrial event is not sensed during the A3 or A4 window, a ventricular pacing escape interval may expire (if a ventricular sensed event signal is not received) resulting in a non-AVS pacing pulse being delivered by pulse generator 202. Control circuit 206 may perform various analyses of the ventricular event data, motion signal data, and in some cases other patient-related history accumulated in memory 210 for determining a cause of missed or undersensed atrial event signals at blocks 306, 308 and/or 310, e.g., using techniques described below in conjunction with FIGs. 9-12.

Slow atrial rate criteria, fast atrial rate criteria, and low atrial event signal amplitude criteria, applied by control circuit 206 at respective blocks 306, 308 and 310, may each include one or more thresholds, values, ranges or other requirements that are compared to respective data, which may include pacing history data, motion signal amplitude data, and/or days since implant of pacemaker 14 for determining if the respective criteria are met. The criteria may be defined to be mutually exclusive to enable control circuit 206 to systematically rule out at least two, and in some instances all three, possible causes of the low AVS pacing condition. The comparisons made by control circuit 206 at each of blocks 306, 308 and 310 may include comparisons that are positive indicators and/or negative indicators of the condition being tested for, e.g., slow atrial rate, fast atrial rate, or low atrial event signal amplitude, for either confirming or ruling out the possible cause of low AVS pacing incidence.

While a particular order of analyzing slow atrial rate criteria, followed by fast atrial rate criteria, followed by low atrial event signal amplitude criteria is shown in FIG. 7, it is to be understood that control circuit 206 may perform the analyses of each set of criteria in a different order or in simultaneous, parallel or integrated processing and decision-making manner that leads to at most one of decision blocks 306, 308 or 310 having an affirmative ("yes") result. In various examples, the criteria applied first may be known as a most common cause of a low AVS pacing condition, either in the specific patient implanted with pacemaker 14 or based on data from a population of patients. Criteria applied second may correspond to a second most common cause, and criteria applied third may correspond to the third most common cause and so on.

In this way, the processing burden for determining the most likely cause of a low AVS pacing condition may be minimized by targeting the analysis toward a most common cause of a low incidence of AVS pacing pulses, particularly during an atrial synchronous ventricular pacing mode. Furthermore, while three causes of a low incidence of AVS pacing pulses are described herein, it is to be understood that other or additional potential causes of a low AVS pacing condition could be identified and evaluated based on cardiac signal data and/or ventricular event history and/or other patient history. In some cases, user programmed atrial event sensing parameters may be improperly selected and adjustments made to operating parameters using the techniques disclosed herein may improve a low AVS condition due to sub-optimal user-programmed operating parameters.

If none of the analyses performed at blocks 306, 308 or 310 result in an affirmative "yes" result, control circuit 206 may return to block 302 and wait for the next monitoring period to end. A low AVS pacing condition may be determined again after another monitoring period and more recent data may provide an affirmative result of the criteria being met for determining one of the causes of the low AVS pacing conditions at block 306, 308 or 310.

In some instances, after determining a cause of a low AVS pacing condition, a low AVS pacing condition may be determined again by control circuit 206 during the next monitoring period. The determined cause of the low AVS pacing condition may be the same or a different cause. For example, both a slow (or fast) atrial rate and a low atrial event signal amplitude may be present causing a low AVS pacing condition. The low atrial event signal amplitude may be determined as a cause of a low AVS pacing condition and one or more operating parameters may be adjusted based on the determined cause according to the techniques disclosed herein. After operating parameter adjustments are made by control circuit 206, the low AVS pacing condition may be determined again by control circuit 206, e.g., after the next monitoring period. A different cause, e.g., a fast atrial rate or a slow atrial rate may be determined as the cause of the low AVS pacing condition the second time it is detected such that control circuit 206 may make additional operating parameter adjustments. In other examples, a combination of criteria applied at blocks 306, 308 and 310 may be applied by control circuit 206 such that both a slow or fast atrial rate and a low atrial event signal amplitude may be determined as a cause of a low AVS pacing condition. Control circuit 206 may therefore determine a cause of a low AVS condition to be at least one of a slow atrial rate or a fast atrial rate and/or low atrial event signal amplitude in some examples.

When criteria for determining one of the causes of the low AVS pacing condition is met at one of blocks 306, 308 or 310, control circuit 206 may select one or more operating control parameter settings at respective block 312, 314 or 316 based on the determined cause of the low incidence of AVS pacing pulses. One or more operating control parameters may be adjusted from a current setting to the new, selected setting to be applied in controlling pacemaker functions during the next monitoring time period. The prior operating control parameter may be stored in memory 210, with related ventricular event data and/or motion signal data associated with the prior operating control parameter setting. The historical data may be used by control circuit 206 for troubleshooting a subsequent, future determination of low AVS pacing condition in some examples. Control circuit 206 may operate according to the operating control parameter(s) selected at one of blocks 312, 314 or 316 for controlling ventricular pacing. The operating control parameters that may be selected may include control parameters that are used for sensing atrial event signals used in setting AV pacing intervals for controlling ventricular pacing. The operating control parameters that may be selected may include a ventricular pacing lower rate, a pacing rate smoothing increment, and/or a pacing mode depending on the determined cause of the low AVS pacing condition. Various examples of operating control parameter settings that may be selected at one of blocks 312, 314 or 316 are described below.

FIG. 8 is a flow chart 350 of a method for accumulating ventricular event data and motion signal data in memory 210 for use in determining and troubleshooting a low AVS pacing condition according to some examples. The process of flow chart 350 may correspond to block 302 of FIG. 7 for acquiring ventricular event data and motion signal data. Data may be acquired for assessing the incidence of AVS pacing pulse delivery and troubleshooting a low AVS pacing condition.

At block 352, control circuit 206 may start a predetermined monitoring period. The monitoring period may be several minutes, several hours, or several days in various examples. The monitoring period may be 24 hours in an example. The monitoring period may be the same for all data being accumulated in memory 210 for detecting and troubleshooting a low AVS pacing condition. In other examples, some data, e.g. ventricular event related data, may be acquired over one monitoring period and other data, e.g., sensed A4 signal related data and/or other motion signal related data, may be acquired over one or more different monitoring time periods, which may have the same or a different time duration than the monitoring period over which ventricular event data is acquired. In some examples, motion signal data may be acquired from a portion of the monitoring period that ventricular event data is acquired. For the ease of illustration, the method of flow chart 350 assumes that ventricular event data and motion signal data are determined and accumulated in memory 210 over the same monitoring period, e.g., a 24 hour monitoring period.

At block 354, control circuit 206 may log ventricular (V) events in memory as they occur. For example, each ventricular pacing pulse delivered by pulse generator 202 can be counted in memory and logged as either an AVS pacing pulse or a non-AVS pacing pulse. A ventricular cycle length (VCL), which is the time interval from a most recent preceding ventricular event (sensed or paced) to the current ventricular event (sensed or paced), may also be logged in memory 210 in conjunction with the logged V event type.

It is noted that in addition to counting ventricular pacing pulses by type (either AVS pacing pulses or non-AVS pacing pulse), control circuit 206 may log any intrinsic ventricular sensed events, based on Vsense signals received from sensing circuit 204 (see FIG. 4). In some cases, relatively few or no ventricular sensed events may be logged because a patient receiving pacemaker 14 may have total AV conduction block. When a Vsense is logged, control circuit 206 may track a count of the Vsense signals that occur following a sensed A4 signal and a count of the Vsense signals that occur following a preceding ventricular event, without an intervening sensed A4 signal, and may log the VCL in conjunction with the logged ventricular sensed event. In some examples, a threshold applied to the percentage of AVS pacing pulses for determining a low AVS pacing condition may be decreased or increased based on the percentage of logged Vsense signals that follow a sensed A4 signal. Some patients may experience intermittent AV conduction such that the percentage of Vsense signals following a sensed A4 signal may increase during periods of AV conduction, resulting in an appropriate decrease in AVS pacing pulse percentage. Accordingly, in some examples, control circuit 206 may determine a low AVS pacing condition based on the percentage of AVS pacing pulses being less than a threshold percentage that is scaled according to a percentage of Vsense events following sensed A4 signals.

By tracking and counting all ventricular events in memory 210, control circuit 206 is enabled to determine percentages of the various ventricular events by type out of the total number of ventricular events logged over the monitoring period. Furthermore, ventricular events logged in memory 210 may include a time stamp to enable control circuit 206 to determine percentages of ventricular events of a particular type, e.g., AVS pacing pulses vs. non-AVS pacing pulses, that occur during a particular subsegment of the monitoring period, e.g., during a particular time of day, over a most recent time period of the total monitoring period, or the like. For example, as described below in conjunction with FIG. 9, control circuit 206 may evaluate the percentage of non-AVS pacing pulses and/or AVS pacing pulses that occur during the night vs. day for use in determining a likely cause of a detected low AVS pacing condition.

At block 356, control circuit 206 may determine and log in memory 210 one or more amplitudes of the motion signal determined during all or a portion of cardiac cycles during the monitoring period as cardiac signal data. A maximum amplitude of the motion signal may be logged for at least some cardiac cycles during the monitoring period that include a sensed A4 signal. For example, control circuit 206 may determine the maximum peak amplitude of the motion sensor signal following an A4 sensing threshold crossing that occurs during either the A3 window or the A4 window. The maximum peak amplitude of the motion sensor signal following an A4 sensing threshold crossing, either in the A3 window or in the A4 window, is referred to as the "A4 signal peak amplitude." Control circuit 206 may determine the A4 signal peak amplitude from the motion sensor signal during the AV pacing interval that extends from the A4 sensing threshold crossing to the ventricular pacing pulse delivered upon expiration of the AV pacing interval. The A4 signal peak amplitudes logged in memory 210 by control circuit 206 may be stored or labeled according to whether the A4 signal was sensed in the A3 window or the A4 window. In this way, control circuit 206 is able to determine a representative A4 signal peak amplitude from A4 signals sensed during the A3 window and/or a representative A4 signal peak amplitude of the A4 signals sensed during the A4 window. The representative amplitude may be a mean, median, mode, nth percentile, maximum, minimum, range, variability or other statistical metric of the maximum peak amplitudes logged in memory 210.

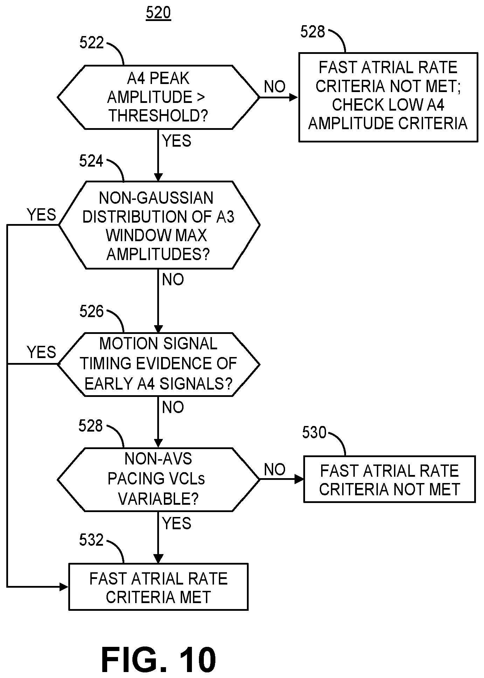

Additionally or alternatively, control circuit 206 may log a maximum amplitude of the motion signal during the A3 window and/or a maximum amplitude of the motion signal during the A4 window, which may be independent of whether or not an A4 event signal is sensed during the cardiac cycle. The maximum amplitude may or may not be greater than or equal to the A4 sensing threshold amplitude. As such, the maximum amplitude determined during an A3 or A4 window may or may not correspond to a sensed A4 signal but may be logged in memory 210 as cardiac signal data. It recognized that some ventricular cycles may not have an A4 window when the motion signal crosses the A4 sensing threshold during the A3 window. As such, control circuit 206 may acquire and store a higher number of A3 window maximum amplitudes than A4 window maximum amplitudes during a given monitoring period. A portion of memory 210 may be allocated to log A3 window and A4 window maximum amplitudes in histogram bins to enable control circuit 206 to analyze a frequency distribution of the maximum amplitudes for troubleshooting a cause of a low AVS pacing condition as described below, e.g., in conjunction with FIG. 10.

Additionally or alternatively, control circuit 206 may log a maximum amplitude of the motion signal determined during the PVAB, or during a portion of the PVAB, as cardiac signal data. A maximum amplitude of the motion signal during the blanking period may correspond to the peak amplitude of the A1 signal or the A2 signal (described above in conjunction with FIGs. 5 and 6). The maximum amplitude of a ventricular event signal (A1 or A2 signal) in the motion signal may be a good indication of the overall signal strength of cardiac event signals in the motion signal. As described below, control circuit 206 may determine when small A4 signals, i.e., A4 signals having a low maximum peak amplitude, may be the most likely cause of a low AVS pacing condition. The relative magnitude of the motion signal during the PVAB may be an indication of the overall cardiac event signal strength and therefore the relative signal strength (amplitude) of the A4 signals. Additionally, a low maximum amplitude of the motion signal during a late portion of the PVAB, e.g., in the last 50 to 100 ms, may indicate that the PVAB can be shortened, e.g., when the cause of the low AVS pacing condition is determined to be a fast atrial rate. Accordingly, control circuit 206 may be configured to determine and log maximum amplitudes of the motion signal determined during the PVAB following each ventricular event.

At block 358, control circuit 206 may determine and store other motion sensor signal related data. In some examples, crossing times of a threshold amplitude by the motion sensor signal that occur during the A3 window and/or during the A4 window may be logged in memory 210. The threshold amplitude may be the A4 sensing threshold amplitude or another threshold amplitude set for enabling control circuit 206 to analyze the amplitude and timing of event signals in the motion signal for use in troubleshooting a low AVS pacing condition. The threshold crossing time in a given cardiac cycle may be determined for a positive-going threshold crossing and/or a negative going threshold crossing. The threshold crossing time in a given cardiac cycle may be determined as the time interval from a ventricular event (paced or sensed) until the threshold crossing time by the motion signal. The threshold crossing times may be determined outside of the PVAB.

For example, the positive-going crossing times of the A4 sensing threshold during the A3 windows and/or during the A4 windows, corresponding to the times of sensed A4 signals and start of the AV pacing intervals, may be stored in memory 210. Additionally or alternatively, in some examples the latest negative-going crossing time of a test threshold amplitude during the A3 window may be stored in memory 210. The test threshold amplitude may be set to a nominal value or to a percentage or portion of the second lower A4 sensing threshold amplitude applied during the A4 window. As described below, control circuit 206 may analyze a frequency distribution of threshold crossing times of the motion sensor signal for determining a cause of a low AVS pacing condition. A portion of memory 210 may be allocated to store threshold crossing times of the motion signal in histogram bins to enable control circuit 206 to analyze a frequency distribution of motion signal threshold crossing times.

When the monitoring period expires, as determined at block 360, control circuit 206 may determine the percentages of ventricular event types out of all ventricular events as described above. For example, the percentage of AVS pacing pulses and the percentage of non-AVS pacing pulses out of the number of all ventricular events counted during the monitoring period may be determined at block 362. At block 364, control circuit 206 may determine representative values, metrics, or other parameters relating to the stored motion signal-related data. For example, control circuit 206 may determine one or more of: a representative A4 signal amplitude for A3 window sensed A4 signals, a representative A4 signal amplitude for A4 window sensed A4 signals, a representative motion signal amplitude during the PVAB, a mode of threshold crossing times by the motion signal during the A3 window and/or a mode of threshold crossing times by the motion signal during the A4 window. Other examples of metrics of the motion signal data that may be determined by control circuit 206, which may be stored in memory 210 as cardiac signal data, following each monitoring period are described below.

Control circuit 206 returns to block 352 to start the next monitoring period when the current monitoring period expires. The next monitoring period may be started immediately upon expiration of the current monitoring period. It is to be understood that control circuit 206 may process data acquired during the monitoring period that just expired after the next monitoring period is started at block 352 for making the various determinations of percentages, metrics or other parameters at blocks 362 and 364 for use in determining a low AVS pacing condition and subsequent determination of a cause of the low AVS pacing condition and a corrective action taken.

It is to be further understood that motion signal metrics may not be determined by control circuit 206 upon expiration of each monitoring period. In some examples, control circuit 206 may evaluate the ventricular event history for detecting a low AVS pacing condition. Control circuit 206 may determine motion signal metrics from motion signal-related data stored during the monitoring period in response to detecting a low AVS pacing condition. Control circuit 206 may determine the motion signal metrics from stored motion signal data on an as needed basis to minimize unnecessary processing time and battery current drain. If a low AVS pacing condition is not detected based on ventricular event data acquired during a monitoring period, the motion signal-related metrics may not be determined by control circuit 206 for that monitoring period.

In FIG. 8, cardiac motion signal data, which may include amplitude and/or timing related data, is determined and stored in memory 210 on a beat-by-beat basis as ventricular event data is logged in memory 210 over the same monitoring period. It is contemplated, however, that some or all motion signal data may be acquired and stored in response to detecting a low AVS pacing condition. Some or all of the motion signal data described herein may be acquired and stored in memory 210 for other pacemaker functions or for providing data for use in diagnostic or manual troubleshooting purposes. In this case, motion signal data may be determined and stored in memory 210 for immediate availability as needed. For example, motion signal data may or may not be acquired by control circuit 206 during a telemetry session with another medical device, e.g., with external device 20 of FIG. 1. In other examples, however, motion signal data, particularly data that is not being acquired for other pacemaker functions or for review by a clinician or technical expert, may be acquired and stored in memory 210 in response to detecting a low AVS pacing condition.

For example, when a low AVS pacing condition is detected according to the example techniques described in conjunction with FIG. 7, control circuit 206 may begin acquiring motion signal data during a post-monitoring period, which may be the next monitoring period or a relatively shorter time period or subsegment of the next monitoring period. The post-monitoring period during which motion signal data is acquired may be one to two minutes long, one to two hours long, several hours long, 24 hours long or any other selected time period that allows control circuit 206 to acquire and store sufficient motion signal data for determining a likely cause of the detected low AVS condition.

FIG. 9 is a flow chart 500 of a method that may be performed by control circuit 206 for determining when slow atrial rate criteria are met according to some examples. The method of flow chart 500 may be performed at block 306 of FIG. 7, for example, to determine when a slow atrial rate is a likely cause of a low AVS pacing condition. A slow atrial rate may be an atrial rate that is less than the programmed ventricular lower rate. Non-AVS ventricular pacing pulses may be delivered upon expiration of ventricular lower rate intervals when the atrial rate is slower than the programmed ventricular lower rate because an A4 signal is not sensed before the lower rate interval expires. Therefore, when the atrial rate is slower than the ventricular lower rate, the non-AVS pacing pulse percentage increases, and the AVS pacing pulse percentage decreases.

Comparisons made at block 306 of FIG. 7 may include one or more of: a comparison of the percentage of non-AVS pacing pulses to a threshold percentage, a comparison of the portion of non-AVS pacing pulses occurring during the night to a threshold percentage, a comparison of a representative A4 signal amplitude to an amplitude threshold, a comparison of the number of days since pacemaker implant to a threshold number of days, and/or a determination of whether the patient has a known history of a relatively higher percentage of AVS pacing pulses when the programmed ventricular pacing lower rate is less than the currently programmed pacing lower rate. The ventricular pacing lower rate is the minimum ventricular pacing rate that is delivered in the absence of sensed atrial event signals and ventricular sensed event signals from sensing circuit 204. One or more of the example comparisons listed above may be made in a variety of combinations. The method of flow chart 500 presents one example of the comparative analysis that control circuit 206 may perform to determine if slow atrial rate criteria are met, which may indicate a most probable cause of a low AVS pacing condition.

At block 502 of FIG. 9, control circuit 206 may determine if a representative amplitude of sensed A4 signal peak amplitudes is greater than a threshold amplitude. The representative amplitude may be determined from A4 signals sensed only during the A4 window in some examples. Control circuit 206 may determine a maximum peak amplitude of the rectified motion signal following an A4 sensing threshold crossing during the A4 window of multiple cardiac cycles during a monitoring period. The maximum peak amplitudes may be stored in memory over a monitoring period of 24 hours (or other monitoring period duration) or for a fewer number of most recent sensed A4 signals, e.g., the most recent 6, 12, 18, 24, 32 or other predetermined number of sensed A4 signals. In still other examples the maximum peak amplitude of A4 signals sensed during the A4 windows may be sampled periodically during a monitoring period. The representative value may be a mean, median, nth percentile, minimum, maximum or other metric of the peak amplitudes of A4 signals sensed during the A4 window.

If the representative amplitude is not greater than the threshold amplitude at block 504 ("no" branch), control circuit 206 may determine that slow atrial rate criteria are not met at block 508 in some examples. Regardless of the atrial rate, when the A4 signal amplitude is very low, e.g., when the representative amplitude is less than or equal to the threshold amplitude applied at block 502, a low A4 signal amplitude may be the more likely cause of the low AVS pacing condition than the atrial rate. The threshold amplitude may be, without limitation, 0.6, 0.7, 0.8, 0.9, 1.0, or 1.1 m/s² as examples. Frequent undersensing of atrial event signals due to the low A4 signal amplitude may result in the low AVS pacing condition. As such, control circuit 206 may determine that slow atrial rate criteria are not met at block 508 when the representative A4 signal amplitude is less than (or equal to) the amplitude threshold. Control circuit 206 may advance to block 310 of FIG. 7 to determine if low A4 signal amplitude criteria are met. The negative result of block 502 does not necessarily rule out a slow atrial rate contributing to a low AVS condition. The actual atrial rate may be slow; however when the A4 signal amplitude is less than the threshold at block 502, the small A4 signals may be a more frequent or more probable cause of undersensed A4 signals and low incidence of AVS pacing pulses.

Referring again to FIG. 9, when the representative A4 signal amplitude is greater than the threshold amplitude at block 502 ("yes" branch), control circuit 206 may advance to block 504. At block 504, control circuit 206 may compare the days since implant to a threshold number of days. At the time of powering on the pacemaker during an implantation procedure, control circuit 206 may begin tracking the time since implant and store the cumulative time in memory 210. If the days since pacemaker implant is determined by control circuit 206 to be greater than a threshold number of days, e.g., greater than 7 days, 10 days, 14 days, 30 days or any other selected number of days, the atrial rate is more likely to be a slow rate than a fast rate. The sinus rate tends to be elevated in some patients during the first one to two post-operative weeks, and sometimes longer, following pacemaker implant. If the AVS pacing frequency is low during the post-operative healing phase, e.g., during the first week to first month after pacemaker implant, a slow atrial rate is less likely to be the cause of a low AVS pacing frequency than a fast atrial rate. As such, in some examples, when control circuit 206 determines that the number of days since implant is not greater than a threshold number of days ("no" branch of block 504), control circuit 206 may advance to block 510 and determine that slow atrial rate criteria are not met. Control circuit 206 may advance to block 308 of FIG. 7 to check if fast atrial rate criteria are met when the number of days since pacemaker implant is less than the threshold number of days.

When the number of days since pacemaker implant is greater than the threshold number of days at block 504, control circuit 206 may determine and compare the percentage of non-AVS pacing pulses to a threshold percentage at block 506. When a low AVS pacing condition is detected at block 304 of FIG. 7, e.g., based on less than a threshold percentage of AVS pacing pulses out of all ventricular events, a high percentage of non-AVS pacing pulses out of all ventricular events may indicate that the atrial rate is slower than the currently programmed ventricular pacing lower rate. Ventricular pacing pulses may be delivered when the lower rate interval (LRI), corresponding to the programmed ventricular pacing lower rate, expires without an atrial event (or ventricular R-wave) being sensed. If the LRI is shorter than the atrial rate interval (e.g., when the programmed lower rate is faster than the intrinsic atrial rate) a high percentage of ventricular pacing pulses will be non-AVS pacing pulses, even during the atrial synchronous ventricular pacing mode. When the non-AVS pacing percentage is greater than a threshold percentage (decision block 506), control circuit 206 may determine that the slow sinus rate criteria are met at block 516.

In some examples, at block 506, control circuit 206 may compare the threshold percentage to a total percentage of non-AVS ventricular pacing pulses determined from the entire duration of the monitoring time period, e.g., 24 hours. Additionally or alternatively, control circuit 206 may determine the percentage of non-AVS ventricular pacing pulses occurring during nighttime hours at block 506. The sinus rate is generally slower at night, when the patient is expected to be resting or asleep, than the sinus rate during the day. A relatively high percentage of non-AVS pacing pulses occurring during nighttime hours, therefore, is a positive indication that a slow atrial rate is a likely cause of the low AVS pacing condition.

Control circuit 206 may be configured to determine the nighttime percentage of non-AVS pacing pulses out of all ventricular events occurring during a one, two, four, six, or eight hour period or any other time period occurring at night, e.g., starting from between 10 p.m. and 3 a.m., or another nighttime hour, and extending for a predetermined time period. Control circuit 206 may compare the nighttime percentage of non-AVS pacing pulses to a predetermined threshold percentage, e.g., a threshold of 10%, 20%, 30%, 40%, 50% or any other specified threshold. Additionally or alternatively, control circuit 206 may compare the nighttime percentage of non-AVS pacing pulses to a daytime percentage of non-AVS pacing pulses (e.g., determined over any other portion of the day, such as between any time period between 8 a.m. to 10 p.m.).

In an example, control circuit 206 may compare the percentage of non-AVS pacing pulses during a nighttime 12 hour period starting from 10 p.m. to a daytime 12 hour period starting from 10 a.m., or another time in the morning selected to divide a 24 hour period into a nighttime period and a daytime period. When the non-AVS pacing pulse percentage during the nighttime 12 hour period is greater than the non-AVS pacing pulse percentage during the daytime 12 hour period, control circuit 206 may determine that the slow atrial rate criteria are met at block 516.

In still other examples, control circuit 206 may compare the nighttime percentage of non-AVS pacing pulses to the total percentage of non-AVS pacing pulses determined from the entire monitoring period, e.g., from an entire 24-hour period. When the nighttime percentage of non-AVS pacing pulses is higher than a threshold, which may be a predetermined threshold percentage, a daytime percentage of non-AVS pacing pulses, and/or a total percentage of non-AVS pacing pulses, control circuit 206 may determine that slow atrial rate criteria are met at block 516. In some examples, control circuit 206 may determine that slow atrial rate criteria are met at block 516 when the total percentage of non-AVS pacing pulses and the nighttime percentage of non-AVS pacing pulses are each greater than a respective threshold. The slow atrial rate is determined as a cause of the low AVS pacing condition.

When the non-AVS pacing percentage is less than or equal to the threshold percentage and/or a nighttime non-AVS pacing percentage is less than or equal to a respective threshold, control circuit 206 may advance to block 512. Control circuit 206 may determine if the patient has a history of a higher percentage of AVS pacing pulses (or a lower percentage of non-AVS pacing pulses) when the programmed ventricular pacing lower rate was programmed to a slower rate than the currently programmed lower rate. As further described below, one corrective action that control circuit 206 may take in response to determining a slow atrial rate is to decrease the currently programmed ventricular pacing lower rate to a slower pacing lower rate. If the patient has a known history of a higher AVS pacing frequency when the ventricular lower rate is programmed to a slower rate than the currently programmed lower rate as determined at block 514, control circuit 206 may determine that the slow atrial rate criteria are met at block 516.

Referring again to block 512, control circuit 206 may determine from a programming history stored in memory 210 if the lower rate is currently at a higher setting (faster ventricular pacing lower rate) than a previously programmed lower rate. In some examples, control circuit 206 may log a history of ventricular events, e.g. percentage of AVS pacing pulses, percentage of non-AVS pacing pulses, percentage of sensed ventricular events and so on, in association with the programmed lower rate in effect at the time of the corresponding ventricular event history. In some examples, other operating parameter values in effect at the time of determined percentages of ventricular events, such as A3 window ending time, the first higher A4 sensing threshold amplitude, the second low A4 sensing threshold amplitude or the like may also be stored in association with determined percentages of ventricular events. Control circuit 206 may be configured to log in memory 210 a history of at least the AVS pacing pulse percentage and associated programmed lower rate(s), e.g., since the time of pacemaker implant. In this way, control circuit 206 may be configured to determine historical values of operating control parameter settings that may be associated with a higher percentage of AVS pacing pulses.

If the programmed lower rate is not faster than any previously programmed lower rate at block 512, control circuit 206 may determine that slow atrial rate criteria are not met at block 510. Control circuit 206 may proceed to block 308 of FIG. 7 to determine if fast atrial rate criteria are met. When control circuit 206 determines that a previously programmed lower rate is less (slower) than the currently programmed lower rate, control circuit 206 may advance to block 514. At block 514, control circuit 206 may determine from historical data stored in memory 210 if a higher percentage of AVS pacing pulses is stored in association with the slower, lower rate. In other examples, control circuit 206 may determine that the patient's ventricular pacing history includes a higher percentage of AVS pacing pulses during a time period of a slower programmed lower rate by receiving a notification from external device 20. If control circuit 206 determines that a history of higher AVS pacing pulse percentage is associated with a slower programmed lower rate ("yes" branch of block 514), control circuit 206 may determine that the slow sinus rate criteria are met at block 516. If not, control circuit 206 determines that slow atrial rate criteria are not met and may advance to block 510 to check if fast atrial rate criteria are met.

Multiple comparisons are shown in FIG. 9 in a particular order and described above for determining when slow atrial rate criteria are met. It is to be understood that the order and combination of comparisons made by control circuit 206 may be different than that shown in FIG. 9. For example, control circuit 206 may determine that slow atrial rate criteria are met based only on the percentage of non-AVS pacing pulses being greater than a threshold percentage. In other examples, control circuit 206 may determine that slow atrial rate criteria are met when the number of days since implant is greater than a threshold number of days and the representative A4 signal amplitude is greater than a threshold amplitude or when the percentage of non-AVS pacing pulses is greater than a threshold percentage. One or more of the comparisons described in conjunction with FIG. 9 may be combined in any combination or order for determining when slow atrial rate criteria are met.

Referring again to FIG. 7, when slow atrial rate criteria are determined to be met at block 306, control circuit 206 may advance to block 312 to make one or more corrective adjustments to operating control parameters in an attempt to increase the relative percentage of delivered AVS pacing pulses. The response of control circuit 206 to determining a low AVS pacing condition with a determined cause being a slow atrial rate may include decreasing the pacing lower rate at block 312. The pacing lower rate is the minimum rate at which ventricular pacing pulses are delivered by pulse generator 202 when an A4 signal is not sensed (and an R-wave is not sensed). By decreasing the pacing lower rate, A4 signals occurring at a slow rate may be more likely to be sensed before a ventricular pacing pulse is delivered, thereby increasing the percentage of AVS pacing pulses.

The lower rate may be decreased by a predetermined decrement, e.g., 5 to 20 beats per minute (bpm) lower than the current setting or to a minimum lower rate setting, which may be 30 or 40 bpm. The lower rate may be reduced to a previous setting known to be associated with a higher AVS pacing percentage. Any time that the lower rate is adjusted to a new setting by control circuit 206 (or in response to a user programming command), the old lower rate setting may be stored in memory 210 along with at least the percentage of AVS pacing pulses and optionally other ventricular event data and/or motion signal data to provide a history of AVS pacing performance of pacemaker 14 in association with the programmed lower rate.

In some examples, the adjustments made by control circuit 206 at block 312 in response to determining a slow atrial rate may include increasing a rate smoothing increment. Control circuit 206 may set a rate smoothing interval (RSI) based on recent VCLs and gradually increase the RSI toward the LRI to avoid an abrupt change in ventricular rate when the A4 signal is not sensed. In some examples, control circuit 206 determines a rate smoothing base interval (RSBI) from the most recent paced VCL. The RSBI may be initialized to the programmed LRI. The RSBI may be compared to the next paced VCL determined by control circuit 206 as the time interval between two consecutive pacing pulses, which may be delivered as AVS pacing pulses or non-AVS pacing pulses. If the RSBI is greater than the next paced VCL, it is decreased by an adjustment interval, e.g., by 8 to 20 ms. If the RSBI is less than the next paced VCL, it may be increased by the adjustment interval. If the RSBI is equal to (or within an adjustment interval) of the most recent paced VCL, it is not adjusted by control circuit 206 and remains at its current value. In this way, control circuit 206 may update the RSBI on each paced VCL to track the actual paced ventricular rate on a beat by beat basis. The RSBI may be adjusted up or down by a relatively small adjustment interval, e.g., 8 to 20 ms, based on the actual VCL(s), so that the RSBI trends toward and closely follows the actual VCLs.

The RSI may be determined by control circuit 206 as the RSBI plus a smoothing increment. The smoothing increment may be adjusted at block 312 by control circuit 206 in response to determining a slow atrial rate. For example, the smoothing increment may be increased from 25 ms to 50 ms, from 50 ms to 100 ms, from 100 ms to 200 ms, or from any current setting of the rate smoothing increment to a maximum rate smoothing increment, which may be between 50 and 250 ms. When the atrial rate is relatively slow, a longer smoothing increment may be used by control circuit 206 to adjust the RSI during atrial synchronous ventricular pacing and/or upon switching to a non-atrial tracking ventricular pacing mode.

In some examples, at block 312, control circuit 206 may additionally or alternatively adjust the A3 window ending time and/or the first, higher A4 sensing threshold amplitude applied to the motion signal during the A3 window. Control circuit 206 may increase the A3 window ending time in response to determining a slow atrial rate because the A4 signal is expected to occur at a relatively long time interval after the ventricular pacing pulse. Control circuit 206 may increase the first, higher A4 sensing threshold amplitude because the true A4 signal is not expected to occur early in the cardiac cycle beginning with a ventricular pacing pulse during a slow atrial rate. Increasing the first, higher A4 sensing threshold amplitude can promote reliable A4 signal sensing if the atrial rate is slow by minimizing the likelihood of A3 event oversensing during the A3 window.

The adjustments to one or more operating control parameters made at block 312 by control circuit 206 in response to determining a slow atrial rate may include one or more adjustments intended to increase the percentage of AVS pacing pulses, e.g., by decreasing the pacing lower rate. Other adjustments made at block 312 may include adjustments that are made because the atrial rate is relatively slow but may not necessarily have an effect on the percentage of AVS pacing pulses. For example, increasing the A3 window ending time may be warranted because the atrial rate is slow, and, as such, the overall VCL is relatively long. Increasing the smoothing increment applied to the RSI, increasing the A3 window ending time and/or increasing the first, higher A4 sensing threshold amplitude applied during the A3 window may not have an immediate or direct effect on the relative percentage of AVS pacing pulses but may be logical adjustments to make due to the relatively long atrial cycle length and subsequently long VCL when the ventricular pacing pulses are properly tracking the slow atrial rate. Such adjustments may improve the overall performance of pacemaker 14 in sensing A4 signals and controlling pacing pulse delivery.

It is to be understood that any adjustments to an operating control parameter that may be made by control circuit 206 in response to any determined cause of a low AVS pacing condition may be made within predetermined limits. For example, a maximum and/or minimum limit may be established for a given operating parameter that is adjustable by control circuit 206 in response to a determined cause of a low AVS pacing condition. In some examples, control circuit 206 may adjust an operating parameter in response to a determined cause of a low AVS pacing condition up to a maximum number of times that the operating parameter is allowed to be adjusted.

When control circuit 206 determines that the slow atrial rate criteria are not met at block 306, control circuit 206 may advance to block 308 to determine if fast atrial rate criteria are met. In some instances, control circuit 206 may advance directly to block 310 to determine if low A4 signal amplitude criteria are met as described above in conjunction with FIG. 9.

FIG. 10 is a flow chart 520 of a method that may be performed by control circuit 206 at block 308 of FIG. 7 for determining if fast atrial rate criteria are met. As described above in conjunction with FIG. 9, control circuit 206 may compare a representative amplitude of the A4 signal peak amplitude to a threshold amplitude to first exclude a low A4 signal amplitude as being the cause of the low AVS pacing condition. When the comparison at block 522 is negative ("no" branch), control circuit 206 may determine that fast atrial rate criteria are not met at block 528 and advance to block 310 of FIG. 7 to check if low A4 signal amplitude criteria are met. When the A4 signal peak amplitude is low, e.g., when a representative A4 signal amplitude is less than a threshold amplitude, the most probable cause of the low AVS pacing condition may be small A4 signals instead of atrial rate, though a fast or slow atrial rate could also be contributing.

When the representative A4 signal amplitude is greater than the threshold amplitude at block 522, small A4 signals are a less probable cause of the low AVS pacing condition. Control circuit 206 may advance to block 524 to check other criteria for positively identifying indications of a fast atrial rate as being a cause for the low AVS condition. At block 524, control circuit 206 may analyze the A3 window maximum amplitudes of the motion signal, which may be stored in an amplitude histogram in memory 210. When A4 signals are being sensed primarily in the A4 window, maximum amplitudes determined during the A3 windows correspond to the maximum peak amplitude of A3 event signals. The frequency distribution of the A3 window maximum amplitudes under these circumstances is expected to be a substantially Gaussian distribution (bell-like curve). During a fast atrial rate, however, A4 events may occur during the A3 window. The A3 event signal and the A4 event signal may fuse forming a higher amplitude signal than either of the A3 event signal or A4 event signal alone. In this case, the frequency distribution of A3 window maximum amplitudes may be non-Gaussian because some maximum amplitudes will be the peak amplitudes of A3 event signals and some maximum amplitudes will be the peak amplitudes of the fused A3 and A4 event signals.

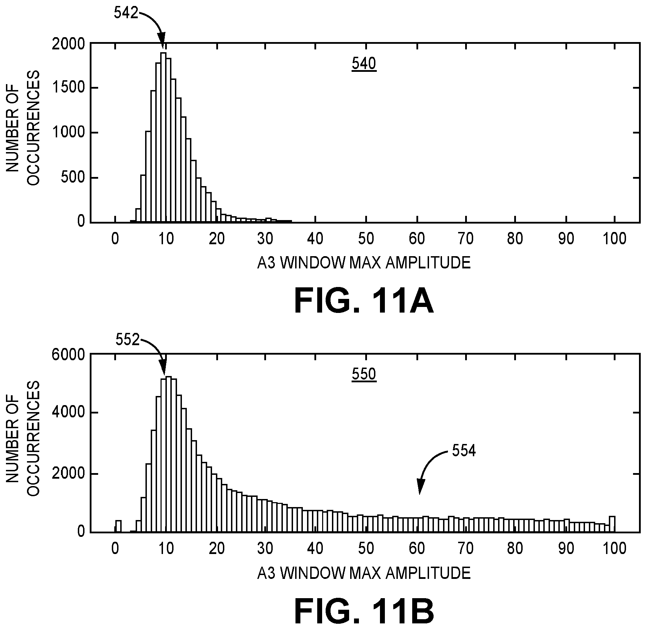

FIG. 11A and FIG. 11B are example histogram plots 540 and 550, respectively, of A3 window maximum amplitudes. The number of occurrences is shown along the y-axis and the A3 window maximum amplitude is shown along the x-axis in ADC units (which can be converted to m/s² by dividing by 10) in both FIG. 11A and 11B. In FIG. 11A, the histogram is a substantially Gaussian distribution having a single peak or mode 542. This substantially normal distribution of A3 window maximum amplitudes is expected during a relatively slow or normal atrial rate, e.g., less than 80 bpm, less than 90 bpm or less than 100 bpm. When the atrial rate is fast, e.g., greater than 80, 90 or 100 bpm, the histogram of A3 window maximum amplitudes will include a higher number of occurrences of relatively higher maximum amplitudes due to fused A3 and A4 event signals occurring during the A3 window.

In FIG. 11B, the frequency distribution of A3 window maximum amplitudes is skewed right. The peak 552 of the histogram is to the left of the center of the range of maximum amplitudes with a right-going tail 554 of high values of maximum amplitudes associated with fused A3 and A4 event signals occurring at relatively lower observation rates than the true A3 signal maximum amplitudes in the histogram bins around peak 552. This example of a non-Gaussian distribution, e.g., a right-skewed distribution, of the A3 window maximum amplitudes is evidence of a fast atrial rate because the right-skewed distribution can be caused by A4 event signals occurring during the A3 window relatively often, causing high amplitude, fused A3 plus A4 event signals.

In other instances, the histogram of A3 window maximum amplitudes may have a bimodal distribution. One (leftmost) peak can be associated with the peak amplitudes of true A3 signals and a second (rightmost) peak can be associated with the higher peak amplitudes of fused A3 and A4 event signals. Accordingly, a bimodal distribution is another example of a non-Gaussian distribution that can be evidence of a fast atrial rate.

Returning to FIG. 10, control circuit 206 may determine that a distribution of A3 window maximum amplitudes is non-Gaussian at block 524 based on a populated A3 window maximum amplitude histogram allocated in memory 210. Multiple histogram bins, each assigned to an amplitude range, spanning a total range of maximum motion signal amplitudes expected during the A3 window may be allocated in memory 210. The histogram may be populated as maximum amplitudes that are determined from A3 windows during the monitoring period. Control circuit 206 may determine a non-Gaussian distribution of the A3 window maximum amplitudes by counting the number of occupied histogram bins, determining a range of occupied histogram bins, or determining a difference between the mode and the greatest maximum amplitude bin that is occupied as examples. When the number of occupied histogram bins, range of occupied histogram bins and/or difference between the mode and the greatest maximum amplitude occupied bin is/are greater than a respective threshold, control circuit 206 may determine that the A3 window maximum amplitude distribution is non-Gaussian. In other examples, control circuit 206 may additionally or alternatively determine the number of occupied bins having greater than a threshold number of occurrences for identifying a bimodal or other non-Gaussian distribution. It is recognized that control circuit 206 may be configured to identify a bimodal, right-skewed or other non-Gaussian distribution of the A3 window maximum amplitudes according to any of a number of different techniques. When control circuit 206 identifies a non-Gaussian distribution at block 524, control circuit 206 may determine that fast atrial rate criteria are met at block 532. A relatively fast atrial rate may be the most probable cause of a low AVS pacing condition.

At block 526, control circuit 206 may analyze motion signal data relating to the timing of threshold crossings by the motion signal during the ventricular cycles. When a patient has a persistently fast atrial sinus rate, A4 signals may be sensed relatively more frequently during the A3 window than when the atrial rate is relatively slow. In a patient having a relatively fast atrial rate, A4 signals that are sensed during the A4 window are more likely to be sensed early in the A4 window rather than relatively late in the A4 window. Accordingly, A4 sensing threshold crossing times by the motion signal may be buffered in memory 210, e.g., according to any of the examples described above in conjunction with FIG. 8. The A4 sensing threshold crossing times may be analyzed by control circuit 206 at block 524 for determining evidence of early A4 signals indicative of a fast atrial rate.

In one example, control circuit 206 may determine if a count of A4 signals sensed during the A3 window is greater than a threshold number or much greater than the number of A4 signals sensed during the A4 window. For example, if the percentage of A4 signals sensed during the A3 window out of all sensed A4 signals is greater than a threshold percentage, e.g., greater than 30%, greater than 40% or greater than 50% as examples, control circuit 206 may determine positive evidence of early A4 signals at block 526 ("yes" branch) and determine that the fast atrial rate criteria are met at block 532.

In another example, control circuit 206 may determine that the number of A3 window maximum amplitudes is greater than the number of A4 window maximum amplitudes stored in memory 210 during the monitoring period. For instance, an A4 window maximum amplitude histogram may have a smaller total number of binned values (fewer observations) than the A3 window maximum amplitude histogram. When the A4 signal is sensed before the A3 window ending time, the A4 window is not started. As such, a maximum amplitude of the motion signal during the A4 window cannot be determined. A larger number of observations in the A3 window maximum amplitude histogram than in the A4 window maximum amplitude histogram is an indication that more A4 signals were sensed during the A3 window than during the A4 window. Therefore, control circuit 206 may determine positive evidence for a fast atrial rate at block 526 when the total number of binned values in the A3 window maximum amplitude histogram is greater than the total number (or greater than a predetermined percentage) of binned values in the A4 window maximum amplitude histogram.

In another example, control circuit 206 may determine if a count of A4 signals sensed in the A4 window occur within a threshold time interval of the A3 window ending time, e.g., within the 50 to 150 ms of the A3 window ending time. In yet another example, control circuit 206 may determine the sum of a count of A4 signals sensed in the A3 window and a count of A4 signals sensed early in the A4 window as a total count of "early" A4 signals. Control circuit 206 may compare this total count of early A4 signals to a threshold number. In some examples, control circuit 206 may determine the count of A3 window sensed A4 signals, the count of early A4 window sensed A4 signals and/or the count of both A3 window and early A4 window sensed A4 signals as a percentage of all A4 signals sensed during the monitoring period (or a predetermined portion thereof). The percentage of early A4 signals sensed during the A3 window, during the early portion of the A4 window, and/or a combination of both may be compared to a respective threshold percentage to determine positive evidence of early A4 signals at block 526.

Various examples of threshold crossing times that may be determined and stored in memory 210 are described above and in conjunction with FIG. 8. When representative threshold crossing times or a determined percentage of threshold crossing times are shorter than a threshold interval, a fast atrial rate may be present. Control circuit 206 may determine motion signal timing evidence of early A4 event signals at block 526 and determine that fast atrial rate criteria are met based on the timing evidence of early A4 event signals at block 532. Control circuit 206 may determine the cause of the low AVS pacing condition as being a fast atrial rate based on at least the sensed A4 signal times (e.g., A4 sensing threshold crossing times) meeting early atrial event criteria, which may be defined as a minimum proportion of sensed A4 signals being sensed within a threshold time interval from the most recent preceding ventricular event.

Additionally or alternatively, control circuit 206 may determine fast atrial rate criteria are met based on the variability of VCLs ending with non-AVS pacing pulses. At block 528, control circuit 206 may analyze the VCLs stored in memory 210 for non-AVS pacing pulses logged during the monitoring period. When the atrial rate is slow, the VCLs of non-AVS pacing pulses will frequently occur at the LRI. The variability of VCLs ending with non-AVS pacing pulses during a slow atrial rate is therefore expected to be relatively small. When the atrial rate is fast, non-AVS pacing pulses may be delivered at rate smoothing intervals to avoid an abrupt change to the pacing lower rate. The VCLs ending with non-AVS pacing pulses delivered at rate smoothing intervals, which may trend toward the LRI, will have a higher variability than pacing pulses delivered at the LRI. As such, the VCLs ending with non-AVS pacing pulses are expected to be more variable during a fast atrial rate than the VCLs ending with non-AVS pacing pulses during a relatively slow atrial rate.

At block 528, control circuit 206 may determine the variance of non-AVS pacing pulse VCLs, the range of VCLs stored for non-AVS pacing pulses, the number of occupied bins in a histogram of VCLs ending with non-AVS pacing pulses stored in memory 210. If the variance, range and/or number of occupied bins or other measure of spread of the non-AVS pacing pulse VCLs is greater than a respective threshold, control circuit 206 may determine that the VCLs ending with non-AVS pacing pulses are variable and determine that fast atrial rate criteria are met at block 532. A number of methods may be conceived and implemented for execution by control circuit 206 for determining the variability of VCLs ending with non-AVS pacing pulses and whether the variability meets criteria or a threshold indicative of a fast atrial rate. Control circuit 206 may determine the most probable cause of the low AVS pacing condition as being a fast atrial rate based on at least the VCLs meeting the variability criteria.

Any of the example criteria described in conjunction with blocks 524, 526 and/or 528 may be used alone or in any combination for determining when fast atrial rate criteria are met by control circuit 206. In some examples, when one criteria is met, e.g., a high percentage of A4 signals sensed during the A3 window, the fast atrial rate criteria may be met at block 532. In other examples, a combination of at least two or more criteria may be required to be met to determine a fast atrial rate as being the cause of a low AVS pacing condition. For instance, a non-Gaussian distribution of A3 window maximum amplitudes (as determined at block 524) and a threshold variability of VCLs ending with non-AVS pacing pulses (as determined at block 528) may both be required to determine that fast atrial rate criteria are met at block 532.

While not shown in FIG. 10 but as described above in conjunction with FIG. 9, the number of days since pacemaker implant may be compared to a threshold number of days by control circuit 206 as part of a method for determining a cause of a low AVS pacing condition. When the number of days since pacemaker implant is less than 7, less than 14, less than 21, less than 30 or any other selected threshold number of days, alone or in combination with any of the example criteria described above in conjunction with FIG. 10, control circuit 206 may determine that fast atrial rate criteria are met.

Referring again to FIG. 7, when the fast atrial rate criteria are met at block 308, control circuit 206 may advance to block 314 to select one or more operating control parameter settings to promote a higher percentage of AVS pacing pulses during the next monitoring period. In some examples, control circuit 206 may select a shorter A3 window ending time than the A3 window ending time setting currently in effect (unless the A3 window ending time is already at a minimum limit). Additionally or alternatively, control circuit 206 may select a lower setting for the first, higher A4 sensing threshold amplitude applied to the motion signal during the A3 window. Because A4 signals can be during the A3 window or early in the A4 window during a fast atrial rate, decreasing the first, higher A4 sensing threshold amplitude and/or shortening the A3 window ending time can promote reliable sensing of the A4 signals coming at relatively short time intervals after a ventricular event. Control circuit 206 may adjust the current setting in effect to the new setting selected in response to determining that fast atrial rate criteria are met and operate according to the adjusted control parameter setting during the next monitoring period.

The first, higher A4 sensing threshold amplitude may be adjusted by decreasing the current setting by a decrement of 0.1 to 1.0 m/s², as examples. The first, higher A4 sensing threshold amplitude may be initially set between 2.5 to 5 m/s², as illustrative examples, and be decreased by a predetermined decrement or percentage or to the next lower available setting, but not less than a minimum threshold amplitude limit, in various examples.

The A3 window ending time may be decreased by 20 to 150 ms as examples and may be decreased by 50, 75 or 100 ms in illustrative examples. The A3 window ending time may be initially set in the range of 500 to 900 ms or in the range of 600 to 800 ms as examples. The A3 window ending time may be decreased by a predetermined decrement or percentage or to the next lower available setting in various examples, but not less than a minimum A3 window ending time limit.

When control circuit 206 decreases the A3 window ending time, which can be set to a time relative to the most recent preceding ventricular event, sensed or paced, the overall A3 window duration may be shortened. For example, the A3 window may be 750 ms in duration, starting from the expiration of the PVAB to the A3 window ending time. If the A3 window ending time is decreased by 50 ms, the overall duration of the A3 window may be shortened by 50 ms to be 700 ms in duration. It is contemplated, however, that control circuit 206 may shorten the PVAB in response to a fast atrial rate determination in some examples. The systolic time interval, e.g., from the A1 signal to the A2 signal or from a ventricular pacing pulse or sensed R-wave to the A2 signal, may be shorter when the atrial rate is relatively fast compared to the systolic time interval when the atrial rate is relatively slower. As such, control circuit 206 may shorten the PVAB at block 314 in response to the fast atrial rate criteria being met at block 308 in some examples. The PVAB may be 500 to 600 ms long as examples and may be shortened by 10 to 100 ms in various examples. Control circuit 206 may determine a maximum amplitude during a latest portion of the PVAB during one or more ventricular cycles, e.g., in the last 50 to 100 ms of the PVAB to verify that the motion signal amplitude is less than a threshold amplitude to confirm that the PVAB can be safely shortened without introducing oversensing of ventricular event signals (e.g., A2 signals as shown in FIG. 5) as false A4 signals. The threshold amplitude applied to the motion signal amplitude during the latest portion of the PVAB period may be less than or equal to the first, higher A4 sensing threshold amplitude applied during the A3 window.

When the PVAB is shortened, the duration of the A3 window may be kept the same by also decreasing the A3 window ending time to occur earlier in the cardiac cycle. In other examples, if the PVAB is shortened, the A3 window duration may or may not be kept the same. When the PVAB is shortened, the A3 window ending time may be decreased or kept the same, e.g., if the first, higher A4 sensing threshold amplitude is decreased instead of shortening the A3 window ending time. As such, the overall duration of the A3 window may increase, decrease or stay the same if the PVAB is decreased, depending on whether or not the A3 window ending time is also adjusted and by how much.

Additionally or alternatively, control circuit 206 may adjust the rate smoothing increment at block 314. As described above, an RSI may be set as the sum of a RSBI that tracks that most recent VCL plus a rate smoothing increment. When fast atrial rate criteria are met at block 308, control circuit 206 may decrease the rate smoothing increment such that the RSI trends toward the LRI more slowly, e.g., over a greater number of ventricular cycles when A4 signals are not being sensed. When A4 signals are sensed intermittently, a shorter rate smoothing increment can maintain the ventricular rate closer to the relatively fast underlying atrial rate such that the A4 signals occurring at regular times during the VCL may be more likely to be sensed. In addition, the inherent atrial rate variability is relatively low when the atrial rate is relatively fast. A shorter rate smoothing increment enables less VCL variability during intermittent A4 undersensing and promotes sustained AV synchrony. Decreasing the rate smoothing increment at block 314 may promote a higher percentage of AVS pacing pulses during the next monitoring period by promoting less variable VCLs, which may in turn promote regular A4 signal sensing. The rate smoothing increment may be initially set to be 50 to 150 ms and may be decreased by 40 to 100 ms by control circuit 206 at block 314. For example, when fast atrial rate criteria are met, the rate smoothing increment may be set in the range of 10 to 50 ms.

In some examples, control circuit 206 may increase the pacing lower rate at block 314 in response to determining that fast atrial rate criteria are met at block 308. In patients having a resting atrial sinus rate that is relatively high, e.g., 90 bpm, 100 bpm or higher, which can sometimes be observed at least in the early post-operative phase, the pacing lower rate may be increased to a faster rate, e.g., 60, 70 or 80 bpm, to provide ventricular rate support at a higher rate. The increase in pacing lower rate may not have a direct impact on increasing the percentage of AVS pacing pulses during the next monitoring period but may be indicated in patients having a normally high, resting atrial rate. For example, instead of a nominal pacing lower rate of 40 to 60 bpm, the pacing lower rate may be increased to a setting of 60 to 70 bpm or up to a predetermined maximum pacing lower rate.

Referring again to block 308, when fast atrial rate criteria are not met, control circuit 206 may advance to block 310 to evaluate the low A4 amplitude criteria. As described above in conjunction with FIGs. 9 and 10, in some examples control circuit 206 may advance directly to block 310 when a representative amplitude of the A4 signal amplitude is less than a threshold (see, for example, block 502 in FIG. 9 or block 522 of FIG. 10). In other examples, control circuit 206 may arrive at block 308 after determining that positive indicators of a slow atrial rate and/or positive indicators of a fast atrial rate do not meet respective slow atrial rate criteria and/or fast atrial rate criteria at blocks 306 or 308, respectively.

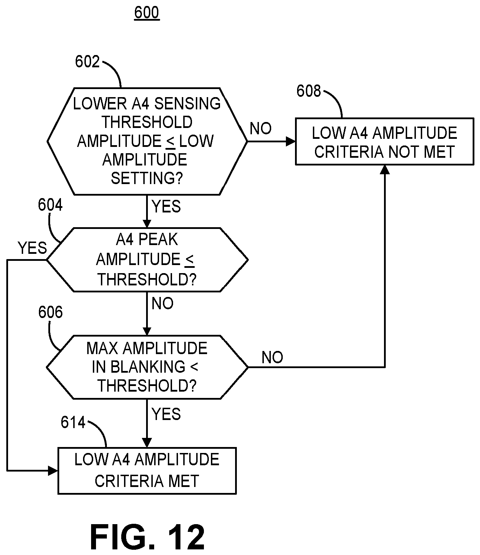

FIG. 12 is a flow chart 600 of a method that may be performed by control circuit 206 for determining when low A4 amplitude criteria are met. The process of flow chart 600 may be performed at block 310 of FIG. 7, for example. At block 602, control circuit 206 may determine if the second, lower A4 sensing threshold amplitude applied to the motion signal during the A4 window is set to a relatively low or minimum amplitude setting. A low or minimum amplitude setting may be in the range of 0.6 to 1.0 m/s² as examples. The second, lower A4 sensing threshold amplitude may be automatically adjusted by control circuit 206 based on the maximum peak amplitude of sensed A4 signals, e.g., as generally disclosed in the above-incorporated U.S. Patent Application No. 17/159,596 (Sheldon, et al.) and in U.S. Patent Application No. 17,159,635 (Sheldon, et al.). The lower A4 sensing threshold amplitude applied during the A4 window is therefore expected to be adjusted to a relatively low or minimum amplitude setting when the sensed A4 signal is relatively small, having a low peak amplitude.

As such, a low or minimum setting of the lower A4 sensing threshold amplitude is an indication of small A4 signals, some of which may be undersensed. Thus, a low or minimum setting of the lower A4 sensing threshold amplitude can be an indication that low A4 signal amplitude is a probable cause of the low AVS pacing condition. In some examples, control circuit 206 may compare the current lower A4 sensing threshold amplitude to a minimum amplitude setting (or second lowest setting or other selected threshold setting) at block 602. If the lower A4 sensing threshold amplitude is greater than the minimum (or other predetermined threshold) setting, e.g., greater than or equal to 1.0 m/s², greater than 0.9 m/s² or greater than 0.8 m/s², control circuit 206 may determine that low A4 amplitude criteria are not met at block 608.

In some examples, when the lower A4 sensing threshold that is applied during the A4 window has been adjusted down to the minimum setting (or less than or equal to another specified amplitude setting), control circuit 206 may determine that low A4 amplitude criteria are met at block 614 based only on the level of the lower A4 sensing threshold amplitude, particularly when other slow atrial rate criteria and fast atrial rate criteria have not been met (at blocks 306 and 308 of FIG. 7). In other examples, control circuit 206 may require that the sensed A4 signal amplitudes are small and that the lower A4 sensing threshold is less than or equal to a low amplitude setting. As such, as shown in the example of FIG. 12, control circuit 206 may advance to block 604 to compare a representative amplitude of the sensed A4 signal peak amplitudes to a threshold amplitude.

As described above, in some examples, control circuit 206 may compare a representative amplitude of the A4 signal peak amplitudes to a threshold amplitude at block 306 and/or block 308 of FIG. 7 (e.g., as described in conjunction with block 502 of FIG. 9 and/or block 522 of FIG. 10) before evaluating positive indicators of a slow atrial rate and/or a fast atrial rate. When the representative amplitude of the sensed A4 signals is not greater than a threshold amplitude, control circuit 206 may advance to the process of flow chart 600. As such, the result of the comparison at block 604 may already be known by control circuit 206 and the analysis need not be repeated.

In some examples, however, the threshold amplitude applied to a representative amplitude of the sensed A4 signals at block 604 may be different than the threshold amplitude applied at blocks 502 and/or 522 of FIGs. 9 and 10, respectively. The threshold amplitude applied at blocks 502 and/or 522 may be a lower amplitude, e.g., 0.7 to 1.0 m/s², than the threshold amplitude applied at block 604, which may be 0.8 to 1.1 m/s² as illustrative, non-limiting examples.

When the lower A4 sensing threshold amplitude is at a low or minimum setting based on the comparison at block 602 and the representative A4 signal amplitude is less than or equal to a threshold amplitude ("yes" branch of block 604), control circuit 206 may advance to block 614 and determine that low A4 amplitude criteria are met. When the representative A4 signal amplitude is not less than or equal to the threshold amplitude at block 604, control circuit 206 may advance to block 606 to evaluate other motion signal data and/or pacing history data for determining if low A4 amplitude criteria are met.

At block 606, control circuit 206 may analyze motion signal maximum amplitudes determined during the PVAB. The maximum amplitude of the motion signal during the PVAB may correspond to the peak amplitude of the A1 or A2 event signals, e.g., as described above in conjunction with FIG. 6. If a representative amplitude of the PVAB maximum amplitudes of the motion signal is less than a threshold amplitude at block 606, control circuit 206 may determine low A4 amplitude criteria are met at block 614. The maximum amplitude of the motion signal during the PVAB may be used as an indication of the overall cardiac event signal strength in the motion signal. If the representative PVAB maximum amplitude is less than the threshold at block 606, the A4 event signals may be likely to have a small amplitude as well and may be frequently oversensed.

When none of the comparisons at blocks 604, 606 or 614 are true, control circuit 206 may determine that low A4 amplitude criteria are not met at block 608. It is to be understood that control circuit 206 may determine that low A4 amplitude criteria are met in response to any one of the example criteria described above, alone or in any combination of two or more requirements. For instance, when slow atrial rate and fast atrial rate criteria are not met and the lower A4 sensing threshold amplitude applied to the motion signal after the A3 window is less than or equal to a low amplitude setting at block 602, control circuit 206 may determine that the low A4 signal amplitude criteria are met based on a single requirement. In other examples, any two out of the three comparisons made at block 602, 604 and/or 606 may be required to be met to determine that the low A4 amplitude criteria are met. In still other examples, all three of the comparisons of blocks 602, 604 and 606 may be required to be true in order to determine that low A4 signal amplitude criteria are met. Accordingly, while multiple decision blocks are shown in FIG. 12 in a particular order with particular combinations yielding a determination that the low A4 amplitude criteria are met, it is to be understood that the decision blocks shown in FIG. 12 may be performed in a different sequence and/or some decision blocks may be omitted altogether.

Referring again to FIG. 7, control circuit 206 may adjust one or more operating control parameters at block 316 in response to determining that the low A4 signal amplitude criteria are met at block 310. When the A4 signal amplitude is small, control circuit 206 may select a differing motion signal sensing vector in an attempt to sense a motion signal having a higher A4 signal amplitude. For instance, control circuit 206 may switch to using a combination (e.g., a summation) of all three axis signals received from a three-dimensional accelerometer (if only one or only two axis signals are currently being used for sensing the motion signal). In other examples, a different combination of two accelerometer axis signals may be selected than a current selection of two axis signals.

Additionally or alternatively, at block 316 control circuit 206 may decrease the lower A4 sensing threshold amplitude that is applied to the motion signal during the A4 window. However, the lower A4 sensing threshold amplitude may already be at a relatively low or minimum setting. It may be assumed that the lower A4 sensing threshold amplitude has been appropriately adjusted based on sensed A4 signal peak amplitudes. A lower amplitude setting may not be available or may not improve the low percentage of AVS pacing pulses due to the small A4 signals.

In addition or alternatively to decreasing the lower A4 sensing threshold amplitude, the lower A4 sensing threshold amplitude may be set to a fixed value with automatic adjustment of the A4 sensing threshold disabled. As generally disclosed in the above-incorporated U.S. Patent Application No. 17/159,596 (Sheldon, et al.) and in U.S. Patent Application No. 17,159,635 (Sheldon, et al.), the A4 sensing threshold amplitude may be automatically adjusted by control circuit 206. The A4 sensing threshold amplitude may be adjusted after a predetermined number of ventricular cycles, e.g., 3 to 12 ventricular cycles, based on the maximum peak amplitude of A4 event signals sensed during the predetermined number of ventricular cycles. The automatic adjustment of the A4 sensing threshold amplitude may be disabled by control circuit 206 in response to determining low A4 signal amplitude. Maintaining the lower A4 sensing threshold amplitude at a fixed, relatively low amplitude, e.g., 0.8 to 1.1 m/s², may promote more reliable sensing of low amplitude A4 event signals, particularly when the A4 event signal amplitude is variable.

In some examples, control circuit 206 may increase the pacing lower rate. When the A4 signals are small and A4 signal sensing cannot be improved by changing the motion signal sensing vector (e.g., by changing or increasing the number of accelerometer axis signals being used), the pacing lower rate may be increased by control circuit 206 at block 316 to promote sufficient ventricular rate support when A4 signals are undersensed. For instance, the current pacing lower rate may be set relatively low, between 40 and 50 bpm to promote sensing of A4 signals and a high incidence of AVS pacing pulses. When low amplitude A4 signal criteria are met following a low AVS pacing condition, the pacing lower rate may be increased by 5 to 20 bpm, e.g., to 50 to 65 bpm, to provide sufficient ventricular rate support to the patient when small A4 signals may be undersensed. Additionally or alternatively, control circuit 206 may switch the pacing mode from the atrial synchronous pacing mode, e.g., a VDD(R) pacing mode, to an asynchronous pacing mode, e.g., a VVI(R) pacing mode.

In some instances, when a monitoring period ends and the percentage of AVS pacing pulses is less than a threshold percentage, control circuit 206 may determine that none of the slow atrial rate criteria, the fast atrial rate criteria or the low A4 signal amplitude criteria are met ("no" branch of block 310). In this situation, the low AVS pacing condition may be due to any of a number of factors, such as variable atrial rate, atrial arrhythmia, highly variable A4 signal amplitude, oversensing of R-waves or any combination of factors. In some instances, therefore, the troubleshooting analysis performed by control circuit 206 in response to determining a low AVS pacing condition may not yield a determined cause. Control circuit 206 may return to block 302 to collect ventricular event data and motion signal data during the next monitoring period and redetermine whether the low AVS pacing condition is still present after the next monitoring period. A cause of the low AVS pacing condition, if still present, may be identified after the next monitoring period from the newly acquired ventricular event and cardiac signal data, leading to one or more adjustments to operating control parameters by control circuit 206. In other instances, the low AVS pacing condition determined from data stored during one monitoring period may be caused by a transient condition, and the low AVS pacing condition may not be detected after the next monitoring period.

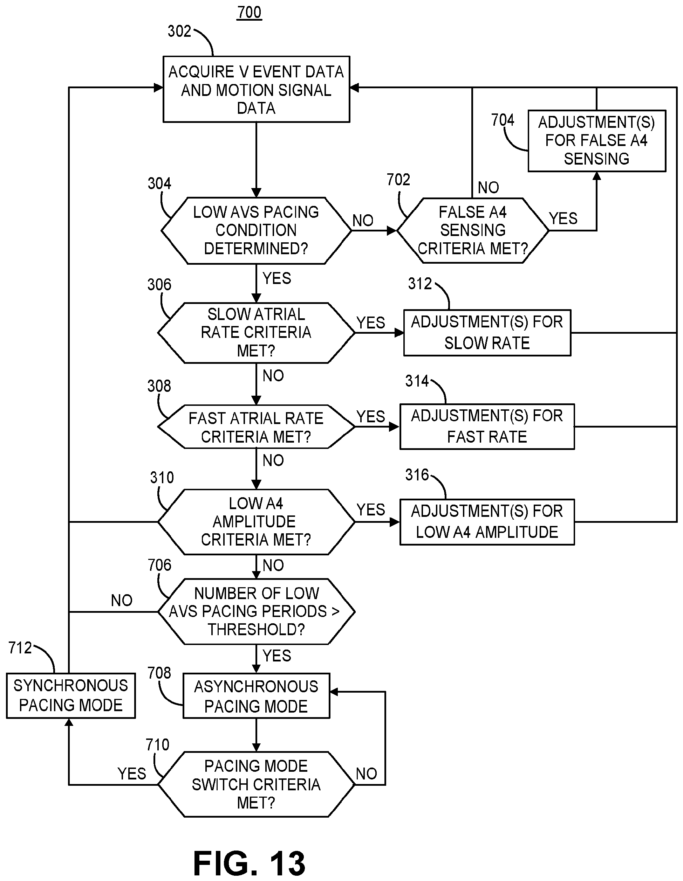

FIG. 13 is a flow chart 700 of a method that may be performed by pacemaker 14 according to another example. Identically numbered blocks in FIG. 13 correspond to like-numbered blocks shown in FIG. 7. As described above in conjunction with the accompanying flow charts and diagrams, pacemaker 14 may be configured to determine when a low AVS pacing condition exists at block 304, e.g., based on less than a threshold percentage of AVS pacing pulses out of all ventricular events (and/or out of all delivered ventricular pacing pulses) occurring during a monitoring period. When the low AVS pacing condition is determined, control circuit 206 may troubleshoot the cause of the low AVS pacing condition and adjust one or more operating control parameters to a different setting to be applied during the next monitoring period for use in controlling ventricular pacing therapy delivery.

In the example of FIG. 13, when a low AVS pacing condition is not determined at block 304 ("no" branch), control circuit 206 may determine if suspected false A4 signal sensing criteria are met at block 702. Oversensing of noise or non-atrial motion signals from the motion signal may cause false A4 signal senses that trigger ventricular pacing pulses delivered at an AV pacing interval, leading to an artificially high AVS pacing pulse count. A high AVS pacing condition may be determined in response to the percentage of AVS pacing pulses being greater than the threshold percentage applied at block 304 for determining a low AVS pacing condition. In other examples, a second higher threshold percentage higher than the threshold percentage applied for determining a low AVS pacing condition may be applied at block 702 for determining a high AVS pacing condition that may be an indication of false A4 signal sensing. For instance, at block 702 control circuit 206 may compare the percentage of AVS pacing pulses to a second, higher threshold percentage, e.g., 80%, 90%, 95%, or 98% as examples, as an indication that AVS pacing may be artificially high due to false A4 signal sensing.

In addition or alternatively to comparing the AVS pacing pulse percentage to a high threshold percentage at block 702, control circuit 206 may perform an analysis of motion signal data at block 702 for determining if false A4 sensing criteria are met at block 702. Control circuit 206 may evaluate a histogram of sample times (e.g., relative to a most recent preceding ventricular event) of sensed A4 signals. When baseline noise in the motion signal is being oversensed, false A4 signals may be frequently sensed early during the A4 window, as soon as the A4 sensing threshold amplitude is decreased from the first, higher sensing threshold amplitude to the second, lower sensing threshold amplitude at the expiration of the A3 window. In other instances, if the A3 window ending time is set too short, the A3 signal may be after the A3 window ending time and be oversensed as a false A4 signal.

Control circuit 206 may determine the time from a ventricular event to a subsequent sensed A4 signal as a V-AS time interval. Control circuit 206 may determine a frequency distribution of the V-AS time intervals at block 702. In some examples, control circuit 206 may determine the percentage of V-AS time intervals that fall after the A3 window ending time and within the first 50 to 100 ms (or other threshold time interval) of the ending time of the A3 window. When a high percentage or proportion of the V-AS time intervals (e.g., greater than 60%, greater than 70%, greater than 80%, greater than 90% or other selected percentage threshold) fall within a threshold time interval after the A3 window ending time, control circuit 206 may determine that false A4 sensing criteria are met at block 702.

In some examples, a random distribution of V-AS time intervals may indicate higher amplitude noise or body motion signals occurring randomly in the motion signal are being falsely oversensed as A4 event signals. Accordingly, in some examples, a variability of the V-AS time intervals may be analyzed for determining that false A4 sensing criteria are met at block 702. For example, control circuit 206 may determine the variance, range, number of occupied histogram bins, or another statistical variability metric of the V-AS time intervals and compare the variability metric to a threshold value.

When false A4 sensing criteria are met at block 702, e.g., greater than a threshold percentage of AVS pacing pulses and/or V-AS time intervals falling predominately during the early portion of the A4 window or being highly variable, control circuit 206 may make one or more operating control parameter adjustments at block 704 to reduce the likelihood of false A4 signal sensing. In some examples, control circuit 206 may increase the lower A4 sensing threshold amplitude applied during the A4 window. Additionally or alternatively, control circuit 206 may increase the A3 window ending time to extend the A3 window to a later time point after the most recent preceding ventricular event so that the first, higher A4 sensing threshold amplitude is applied to the motion signal through a later time point in the cardiac cycle.

After one or more adjustments to operating control parameters used for sensing A4 signals at block 704, or if false A4 sensing criteria are not met at block 702, control circuit 206 returns to block 302. Control circuit 206 acquires ventricular event data and motion signal data during the next monitoring period. Control circuit 206 operates during the next monitoring period according to any operating control parameters adjusted at block 704 for controlling ventricular pacing therapy delivery. If a low AVS pacing condition is determined or false A4 sensing criteria are met after the next monitoring period, control circuit 206 may make additional adjustments to operating control parameters as warranted, e.g., according to any of the examples given herein.

In some instances, control circuit 206 may determine a low AVS pacing condition after each one of multiple monitoring periods. Control circuit 206 may be configured to make adjustments to one or more operating control parameters after each monitoring period as needed based on a cause of the low AVS pacing condition as determined at one of blocks 304, 306 or 308 according to any of the techniques described herein. In some cases, however, a cause of the low AVS pacing condition may not be identified when none of the slow atrial rate criteria (block 306), fast atrial rate criteria (block 308) or low A4 signal amplitude criteria (block 310) are met.

In the example shown in FIG. 13, control circuit 206 may determine at block 706 if a low AVS pacing condition has been determined for a threshold number of monitoring periods. When a low AVS pacing condition is determined for 2, 3, 5, 7, 10, 14 or other selected number of monitoring periods, which may or may not be required to be consecutive, control circuit 206 may switch the pacing mode at block 708 to an asynchronous pacing mode, e.g., a VVI(R) or VDI(R) pacing mode. In some examples, a threshold number of monitoring periods may be required to correspond to a very low AVS pacing condition, e.g., an even lower percentage of AVS pacing pulses than the threshold percentage applied at block 304. For instance, if two or more monitoring periods are associated with an AVS pacing pulse percentage that is less than 50%, less than 40%, less than 30% or less than 20%, control circuit 206 may switch the pacing mode to an asynchronous pacing mode. The switch may be a permanent switch until reprogrammed by a clinician. A notification may be transmitted from pacemaker telemetry circuit 208 to external device 20 to make the patient and/or a clinician aware of the pacing mode change such that operating control parameters can be evaluated and reprogrammed as necessary to reprogram an atrial synchronous ventricular pacing mode and operating control parameters that promote an improved performance of AVS pacing by pacemaker 14.

In other examples, the pacing mode switch made at block 708 may be temporary, e.g., for one or more monitoring periods. Control circuit 206 may set a timer to a maximum time limit for operating in the asynchronous pacing mode. After a maximum time limit in the asynchronous pacing mode expires, determined as pacing mode switch criteria being met at block 710, control circuit 206 may switch back to the atrial synchronous pacing mode at block 712 and return to block 302 to continue acquiring ventricular event and cardiac motion signal data and restart monitoring for a low AVS pacing condition during the atrial synchronous pacing mode (e.g., VDD pacing mode).

In addition or alternatively to setting a maximum time limit for operating in the asynchronous pacing mode, control circuit 206 may acquire motion signal data for determining when atrial synchronous pacing mode switch criteria are met at block 710. During a VVI(R) pacing mode, control circuit 206 may continue to determine motion signal data, e.g., to determine if the cardiac event signal strength in the motion signal improves. For example, without necessarily applying an A4 sensing threshold to the motion signal for sensing A4 signals, control circuit 206 may determine the maximum amplitude of the motion signal during and/or after the PVAB. Control circuit 206 may compare motion signal maximum amplitude data (or a representative maximum amplitude) acquired during the asynchronous pacing mode to a predetermined threshold or to motion signal maximum amplitude data acquired during the monitoring periods determined to be low AVS pacing. Additionally or alternatively, control circuit 206 may determine and store motion signal maximum amplitude data upon switching to the asynchronous pacing mode for use as baseline maximum amplitude data. Control circuit 206 may compare motion signal maximum amplitude data determined at later time points during the asynchronous pacing mode to the baseline maximum amplitude data.

Control circuit 206 may be configured to determine an increasing trend in the maximum amplitude data. Control circuit 206 may determine that the maximum amplitude data from the motion signal at some time point after switching to the asynchronous pacing mode has increased compared to previously acquired maximum amplitude data, indicating a possible increase in cardiac event signal strength in the motion signal. Control circuit 206 may determine that pacing mode switching criteria are met at block 710 in response to determining an increase in maximum amplitude of the motion signal.

In some examples, the pacing mode switch at block 708 is to an asynchronous pacing mode with dual sensing, e.g., the VDI(R) pacing mode, control circuit 206 may continue to set the A3 window ending time and apply the A4 sensing threshold to the motion signal, during both the A3 window and the A4 window. In other examples, control circuit 206 may switch to a single chamber pacing and sensing mode, e.g., VVI(R) at block 708 and periodically switch to a dual chamber sensing mode, e.g., VDI(R) with A4 signal sensing enabled to determine if pacing mode switching criteria are met at block 710. During asynchronous pacing, some A4 signals may randomly occur during the A3 window and/or the A4 window. Control circuit 206 may collect motion signal data that includes maximum peak amplitudes of sensed A4 signals in addition to or alternatively to maximum amplitude data determined during the PVAB and/or after the PVAB (when an A4 sensing threshold amplitude is not crossed).

Control circuit 206 may be configured to determine that the motion signal amplitude has increased compared to previously determined motion signal amplitudes or that the motion signal amplitude is greater than a threshold amplitude. This may occur when an episode of atrial fibrillation or atrial flutter occurs and spontaneously terminates resulting in a period of low A4 signal amplitude followed by a return of sufficient A4 signal amplitude for sensing A4 event signals and delivering AVS pacing pulses. Control circuit 206 may determine that pacing mode switching criteria are met at block 710 in response to determining an increase in motion signal amplitude during the PVAB and/or after the PVAB and/or in response to determining an increase in the maximum peak amplitudes of sensed A4 signals during the asynchronous ventricular pacing mode.

In response to determining an increase in motion signal amplitude during the asynchronous pacing mode, control circuit 206 may switch back to the atrial synchronous pacing mode at block 712. Control circuit 206 may return to block 302 to monitor for a low AVS pacing condition (and in some examples false A4 sensing associated with a high AVS pacing condition) during the atrial synchronous pacing mode.

In the example of FIG. 13, block 706 is shown for the sake of convenience following block 310. In this example, control circuit 206 may determine when a threshold number of monitoring periods result in a low AVS pacing condition after none of the slow atrial rate criteria, fast atrial rate criteria or low A4 signal amplitude criteria are met for the currently expired monitoring period as a condition for switching to the asynchronous pacing mode. It is contemplated however, that control circuit 206 may determine if a threshold number of monitoring periods result in a low AVS pacing condition each time a low AVS pacing condition is determined at block 304, before advancing to blocks 306, 308 and/or 310 for determining the cause as being one of the slow atrial rate, fast atrial rate or low A4 signal amplitude. As such, in some examples, when control circuit 206 determines a low AVS pacing condition at block 304 has been determined for a threshold number of monitoring periods, control circuit 206 may switch to the asynchronous pacing mode at block 708 without determining a cause of the most recently determined low AVS pacing condition. In this way, troubleshooting and control parameter adjustments may be performed a maximum number of times and then control circuit 206 may switch the pacing mode, permanently or temporarily as described above.

While the illustrative examples presented herein refer to atrial event signal sensing from the motion signal, which can be an acceleration signal, it is contemplated that aspects of the low AVS pacing condition determination and troubleshooting methods disclosed herein may be implemented in any ventricular pacemaker configured to deliver atrial synchronous ventricular pacing pulses in response to atrial events sensed from a cardiac signal. The cardiac signal used for sensing atrial events is not limited to being an acceleration signal. The cardiac signal may be an electrical signal, e.g., received from electrodes carried on the pacemaker housing or by a lead coupled to the pacemaker. Atrial P-waves attendant to atrial depolarization may be sensed from the electrical signal for triggering atrial synchronous ventricular pacing pulses. The atrial P-waves may be near field P-waves or far field P-waves depending on the location of the sensing electrodes.

The cardiac signal used for sensing atrial events may be a mechanical signal sensed by a sensor within or on the pacemaker housing or carried by a lead coupled to the pacemaker. Atrial event signals attendant to the contraction of the atria and active ventricular filling due to atrial kick may be sensed from a mechanical cardiac signal, e.g., an acceleration signal, pressure signal, impedance signal (correlated to heart chamber volume), heart sound signal or any other mechanical cardiac signal, for triggering atrial synchronous ventricular pacing pulses.

Furthermore, aspects of the techniques disclosed herein may be implemented in a pacemaker capable of delivering atrial synchronous ventricular pacing without sensing atrial event signals directly from a sensed cardiac signal. For instance, the ventricular pacemaker may be configured to receive atrial event trigger signals from a second medical device that may be co-implanted in the patient and configured to sense atrial event signals from a cardiac signal. The second medical device may sense P-waves or atrial event signals corresponding to atrial mechanical contraction and/or deliver atrial pacing pulses to an atrial chamber. The second medical device may be configured to transmit an atrial event trigger signal to the ventricular pacemaker. In response to receiving the transmitted atrial event trigger signal, the ventricular pacemaker may deliver an AVS pacing pulse. Examples of ventricular pacemakers that may be adapted to perform aspects of the techniques disclosed herein and are configured to receive a transmitted atrial event trigger signal from another medical device are generally disclosed in U.S. Patent No. 10,350,417 (Cao, et al.) and in U.S. Patent No. 11,229,796 (Zhao, et al.).

It should be understood that, depending on the example, certain acts or events of any of the methods described herein can be performed in a different sequence, may be added, merged, or left out altogether (e.g., not all described acts or events are necessary for the practice of the method). Moreover, in certain examples, acts or events may be performed concurrently, e.g., through multi-threaded processing, interrupt processing, or multiple processors, rather than sequentially. In addition, while certain aspects of this disclosure are described as being performed by a single circuit or unit for purposes of clarity, it should be understood that the techniques of this disclosure may be performed by a combination of units or circuits associated with, for example, a medical device.

In one or more examples, the functions described may be implemented in hardware, software, firmware, or any combination thereof. If implemented in software, the functions may be stored as one or more instructions or code on a computer-readable medium and executed by a hardware-based processing unit. Computer-readable media may include computer-readable storage media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPLAs), or other equivalent integrated or discrete logic circuitry. Accordingly, the term "processor," as used herein may refer to any of the foregoing structure or any other structure suitable for implementation of the techniques described herein. Also, the techniques could be fully implemented in one or more circuits or logic elements.

Thus, a medical device has been presented in the foregoing description with reference to specific examples. It is to be understood that various aspects disclosed herein may be combined in different combinations than the specific combinations presented in the accompanying drawings. It is appreciated that various modifications to the referenced examples may be made without departing from the scope of the disclosure and the following claims.

## Claims

1. A medical device comprising:
a control circuit (206) configured to:
determine a low atrioventricular synchronous pacing condition based on ventricular event data;
determine a cause of the low atrioventricular synchronous pacing condition based on at least one of the ventricular event data and cardiac signal data;
select at least one operating control parameter setting based on the determined cause of the low atrioventricular synchronous pacing condition;
operate according to the at least one selected operating control parameter setting; and
a pulse generator (202) controlled by the control circuit to deliver ventricular pacing pulses while operating according to the at least one selected operating control parameter setting.

2. The medical device of claim 1, wherein the control circuit is further configured to determine the low atrioventricular synchronous pacing condition by:
determining from the ventricular event data a percentage of atrioventricular synchronous pacing pulses out of a total number of ventricular events; and
determining that the percentage of atrioventricular synchronous pacing pulses is less than a threshold percentage.

3. The medical device of any of claims 1-2, wherein the control circuit is further configured to determine the low atrioventricular synchronous pacing condition by:
determining from the ventricular event data a percentage of non-atrioventricular synchronous pacing pulses out of a total number of ventricular events; and
determining that the percentage of non-atrioventricular synchronous pacing pulses is greater than a threshold percentage.

4. The medical device of any of claims 1-3 wherein the operating control parameter setting is at least one of:
a pacing lower rate;
a pacing rate smoothing interval increment;
a pacing mode;
an atrial event sensing threshold amplitude;
an atrial event sensing window ending time; or
a post-ventricular atrial blanking period.

5. The medical device of any of claims 1-4, wherein the control circuit is further configured to determine the cause of the low atrioventricular synchronous pacing condition as at least one of a slow atrial rate, a fast atrial rate or a low atrial signal amplitude.

6. The medical device of any of claims 1-5, wherein the control circuit is further configured to:
determine a percentage of non-atrioventricular synchronous pacing pulses from the ventricular event data;
determine that the percentage of non-atrioventricular synchronous pacing pulses is greater than a threshold percentage; and
determine the cause of the low atrioventricular synchronous pacing condition as being a slow atrial rate based on at least the percentage of non-atrioventricular synchronous pacing pulses being greater than the threshold percentage.

7. The medical device of any of claims 1-6, wherein the control circuit is further configured to determine the cause of the low atrioventricular synchronous pacing condition as being a slow atrial rate by at least one of:
determining that a time interval since implant of the medical device is greater than a threshold time interval; and
determining that a pacing lower rate setting used for controlling the pulse generator to deliver the ventricular pacing pulses is faster than a previous pacing lower rate and that the previous pacing lower rate is associated with a higher percentage of atrioventricular synchronous pacing pulses than the low atrioventricular synchronous pacing condition.

8. The medical device of any of claims 1-5 further comprising a sensor (212) for sensing the cardiac signal,
wherein the control circuit is further configured to:
receive the signal;
for each of a plurality of cardiac cycles:
set a time window corresponding to a passive ventricular filling phase; and
determine a maximum amplitude of the cardiac signal during the time window;
determine that a distribution of the maximum amplitudes is non-Gaussian; and
determine the cause of the low atrioventricular synchronous pacing condition as being a fast atrial rate based on at least the non-Gaussian distribution of the maximum amplitudes.

9. The medical device of any of claims 1-5 and 8 further comprising a sensor (212) for sensing the cardiac signal;
wherein the control circuit is further configured to:
receive the cardiac signal;
sense atrial event signals from the cardiac signal;
determine a sensed atrial event time for each of a plurality of the sensed atrial event signals;
determine that the sensed atrial event times meet early atrial event criteria; and
determine the cause of the low atrioventricular synchronous pacing condition as being a fast atrial rate based on at least the sensed atrial event times meeting early atrial event criteria.

10. The medical device of any of claims 1-9, wherein the control circuit is further configured to:
determine from the ventricular event data ventricular cycle lengths corresponding to non-atrial synchronous ventricular pacing pulses delivered by the pulse generator;
determine that the ventricular cycle lengths meet variability criteria; and
determine the cause of the low atrioventricular synchronous pacing condition as being a fast atrial rate based on at least the ventricular cycle lengths meeting the variability criteria.

11. The medical device of any of claims 1-10, further comprising a sensor (212) for sensing the cardiac signal;
wherein the control circuit is further configured to:
receive the cardiac signal;
sense atrial event signals from the cardiac signal;
determine peak amplitudes of the cardiac signal corresponding to the atrial event signals;
determine a representative amplitude of the peak amplitudes is less than a threshold amplitude; and
determine that the cause of the low atrioventricular synchronous pacing condition is a low atrial signal amplitude based on at least the representative amplitude of the peak amplitudes being less than a threshold amplitude.

12. The medical device of any of claims 1-11, wherein the control circuit is further configured to:
determine that an atrial event sensing threshold that is applied to the cardiac signal for sensing atrial event signals is lower than a low amplitude setting; and
determine that the cause of the low atrioventricular synchronous pacing condition is a low atrial signal amplitude based on at least the atrial event sensing threshold being lower than the low amplitude setting.

13. The medical device of any of claims 1-12, wherein the control circuit is further configured to:
determine a representative maximum amplitude from the cardiac signal data;
determine that the representative maximum amplitude is less than a threshold amplitude; and
determine the cause of the low atrioventricular synchronous pacing condition as being a low atrial signal amplitude based on at least the representative maximum amplitude being less than the threshold amplitude.

14. The medical device of any of claims 1-13 further comprising a sensor (212) for sensing the cardiac signal;
wherein the control circuit is further configured to:
receive the cardiac signal;
sense atrial event signals from the cardiac signal according to at least one sensing control parameter;
determine the low atrioventricular synchronous pacing condition based on a first portion of the ventricular event data corresponding to a first monitoring time period;
determine a high atrioventricular synchronous pacing condition based on a second portion of the ventricular event data corresponding to a second monitoring time period different than the first monitoring time period;
determine that false atrial event signal sensing criteria are met based on at least one of the second portion of the ventricular event data and a portion of the cardiac signal data corresponding to the second monitoring period; and
adjust the at least one sensing control parameter in response to determining that the false atrial event signal sensing criteria are met.

15. The medical device of claim 14, wherein the control circuit is further configured to:
set a time window corresponding to ventricular passive filling, the time window having an ending time;
determine a sensed atrial event signal time for each cardiac cycle of a plurality of cardiac cycles;
determine a proportion of the sensed atrial event signal times that occur within a threshold time interval of the ending time of the time window;
determine that the proportion of the sensed atrial event signal times that occur within the threshold time interval of the ending time of the time window is greater than a threshold proportion; and
determine that the false atrial event signal criteria are met based on the proportion of the sensed atrial event signal times that occur within a threshold time interval of the ending time of the time window being greater than the threshold proportion;
and optionally wherein the control circuit is further configured to adjust the at least one sensing control parameter in response to determining that the false atrial event signal sensing criteria are met by at least one of increasing an atrial event sensing threshold amplitude and increasing an ending time of a time window corresponding to ventricular passive filling.

## Patentansprüche

1. Medizinische Vorrichtung, umfassend:
eine Steuerschaltung (206), die ausgelegt ist zum:
Bestimmen eines Zustands mit niedriger atrioventrikulärer synchroner Stimulation basierend auf Ventrikuläres-Ereignis-Daten;
Bestimmen einer Ursache des Zustandes mit niedriger atrioventrikulärer synchroner Stimulation basierend auf mindestens einem von Ventrikuläres-Ereignis-Daten und kardiale Signaldaten;
Auswählen mindestens einer Betriebskontrollparameter-Einstellung basierend auf der bestimmten Ursache des Zustandes mit niedriger atrioventrikulärer synchroner Stimulation;
Arbeiten gemäß der mindestens einen ausgewählten Betriebskontrollparameter-Einstellung; und
einen Impulsgenerator (202), der durch die Steuerschaltung gesteuert wird, um ventrikuläre Stimulationsimpulse zu liefern, während er gemäß der mindestens einen ausgewählten Betriebskontrollparameter-Einstellung arbeitet.

2. Medizinische Vorrichtung nach Anspruch 1, wobei die Steuerschaltung ferner ausgelegt ist, um den Zustand mit niedriger atrioventrikulärer synchroner Stimulation zu bestimmen durch:
Bestimmen, anhand der Ventrikuläres-Ereignis-Daten, eines Prozentsatzes von atrioventrikulären synchronen Stimulationsimpulsen aus einer Gesamtzahl ventrikulärer Ereignisse; und
Bestimmen, dass der Prozentsatz der atrioventrikulären synchronen Stimulationsimpulse geringer als ein Schwellenprozentsatz ist.

3. Medizinische Vorrichtung nach einem der Ansprüche 1-2, wobei die Steuerschaltung ferner ausgelegt ist, um den Zustand mit niedriger atrioventrikulärer synchroner Stimulation zu bestimmen durch:
Bestimmen, anhand der Ventrikuläres-Ereignis-Daten, eines Prozentsatzes von nicht-atrioventrikulären synchronen Stimulationsimpulsen aus einer Gesamtzahl ventrikulärer Ereignisse; und
Bestimmen, dass der Prozentsatz der nicht-atrioventrikulären synchronen Stimulationsimpulse größer als ein Schwellenprozentsatz ist.

4. Medizinische Vorrichtung nach einem der Ansprüche 1-3, wobei die Betriebskontrollparameter-Einstellung mindestens eines der folgenden ist:
eine untere Stimulationsfrequenz;
eine Schrittweite eines Stimulationsfrequenz-Glättungsintervalls;
ein Stimulationsmodus;
eine Erfassungsschwellenamplitude eines atrialen Ereignisses;
eine Endzeit eines atrialen Ereigniserfassungsfensters; oder
eine postventrikuläre atriale Austastperiode.

5. Medizinische Vorrichtung nach einem der Ansprüche 1-4, wobei die Steuerschaltung ferner ausgelegt ist, um die Ursache des Zustandes mit niedriger atrioventrikulärer synchroner Stimulation als mindestens eines von einer langsamen atrialen Frequenz, einer schnellen atrialen Frequenz oder einer niedrigen atrialen Signalamplitude zu bestimmen.

6. Medizinische Vorrichtung nach einem der Ansprüche 1-5, wobei die Steuerschaltung ferner ausgelegt ist zum:
Bestimmen eines Prozentsatzes von nicht-atrioventrikulären synchronen Stimulationsimpulsen aus den Ventrikuläres-Ereignis-Daten;
Bestimmen, dass der Prozentsatz der nicht-atrioventrikulären synchronen Stimulationsimpulse größer ist als ein Schwellenprozentsatz; und
Bestimmen der Ursache des Zustandes mit niedriger atrioventrikulärer synchroner Stimulation als eine langsame atriale Frequenz zumindest basierend darauf, dass der Prozentsatz von nicht-atrioventrikulären synchronen Stimulationsimpulsen größer als der Schwellenprozentsatz ist.

7. Medizinische Vorrichtung nach einem der Ansprüche 1-6, wobei die Steuerschaltung ferner ausgelegt ist, um die Ursache des Zustandes mit niedriger atrioventrikulärer synchroner Stimulation als eine langsame atriale Frequenz zu bestimmen durch mindestens eines von:
Bestimmen, dass ein Zeitintervall seit der Implantation der medizinischen Vorrichtung länger ist als ein Schwellenzeitintervall; und
Bestimmen, dass eine untere Stimulationsfrequenz-Einstellung, die zur Steuerung des Impulsgenerators verwendet wird, um die ventrikulären Stimulationsimpulse abzugeben, schneller ist als eine vorherige untere Stimulationsfrequenz und dass die vorherige untere Stimulationsfrequenz mit einem höheren Prozentsatz von atrioventrikulären synchronen Stimulationsimpulsen verknüpft ist als der Zustand mit niedriger atrioventrikulärer synchroner Stimulation.

8. Medizinische Vorrichtung nach einem der Ansprüche 1-5, ferner umfassend einen Sensor (212) zum Abfühlen des kardialen Signals,
wobei die Steuerschaltung ferner ausgelegt ist zum:
Empfangen des Signals;
für jeden einer Vielzahl von Herzzyklen:
Einstellen eines Zeitfensters, das einer passiven ventrikulären Füllphase entspricht; und
Bestimmen einer maximalen Amplitude des kardialen Signals während des Zeitfensters;
Bestimmen, dass eine Verteilung der Maximalamplituden nicht-gaußförmig ist; und
Bestimmen der Ursache des Zustandes mit niedriger atrioventrikulärer synchroner Stimulation als eine schnelle atriale Frequenz zumindest basierend auf der nicht-gaußförmigen Verteilung der Maximalamplituden.

9. Medizinische Vorrichtung nach einem der Ansprüche 1-5 und 8, ferner umfassend einen Sensor (212) zum Abfühlen des kardialen Signals;
wobei die Steuerschaltung ferner ausgelegt ist zum:
Empfangen des kardialen Signals;
Erfassen von atrialen Ereignissignalen aus dem kardialen Signal;
Bestimmen einer erfassten atrialen Ereigniszeit für jedes einer Vielzahl der erfassten atrialen Ereignissignale;
Bestimmen, dass die erfassten atrialen Ereigniszeiten Kriterien für ein frühes atriales Ereignis entsprechen; und
Bestimmen der Ursache des Zustandes mit niedriger atrioventrikulärer synchroner Stimulation als eine schnelle atriale Frequenz zumindest basierend darauf, dass die erfassten atrialen Ereigniszeiten den Kriterien für ein frühes atriales Ereignis entsprechen.

10. Medizinische Vorrichtung nach einem der Ansprüche 1-9, wobei die Steuerschaltung ferner ausgelegt ist zum:
Bestimmen, anhand der Ventrikuläres-Ereignis-Daten, von ventrikulären Zykluslängen, die nicht-atrialen synchronen ventrikulären Stimulationsimpulsen entsprechen, die durch den Impulsgenerator abgegeben werden;
Bestimmen, dass die ventrikulären Zykluslängen Variabilitätskriterien entsprechen; und
Bestimmen der Ursache des Zustandes mit niedriger atrioventrikulärer synchroner Stimulation als eine schnelle atriale Frequenz zumindest basierend darauf, dass die ventrikulären Zykluslängen den Variabilitätskriterien entsprechen.

11. Medizinische Vorrichtung nach einem der Ansprüche 1-10, ferner umfassend einen Sensor (212) zum Abfühlen des kardialen Signals;
wobei die Steuerschaltung ferner ausgelegt ist zum:
Empfangen des kardialen Signals;
Erfassen von atrialen Ereignissignalen aus dem kardialen Signal;
Bestimmen, dass die Spitzenamplituden des kardialen Signals den atrialen Ereignissignalen entsprechen;
Bestimmen, dass eine repräsentative Amplitude der Spitzenamplituden kleiner als eine Schwellenamplitude ist; und
Bestimmen, dass die Ursache des Zustandes mit niedriger atrioventrikulärer synchroner Stimulation eine niedrige atriale Signalamplitude ist, zumindest darauf basierend, dass die repräsentative Amplitude der Spitzenamplituden kleiner als eine Schwellenamplitude ist.

12. Medizinische Vorrichtung nach einem der Ansprüche 1-11, wobei die Steuerschaltung ferner ausgelegt ist zum:
Bestimmen, dass eine Erfassungsschwelle eines atrialen Ereignisses, die auf das kardiale Signal angewendet wird zum Erfassen von atrialen Ereignissignalen, niedriger als eine niedrige Amplitudeneinstellung ist; und
Bestimmen, dass die Ursache des Zustandes mit niedriger atrioventrikulärer synchroner Stimulation eine niedrige atriale Signalamplitude ist, zumindest darauf basierend, dass die Erfassungsschwelle eines atrialen Ereignisses niedriger als eine niedrige Amplitudeneinstellung ist.

13. Medizinische Vorrichtung nach einem der Ansprüche 1-12, wobei die Steuerschaltung ferner ausgelegt ist zum:
Bestimmen einer repräsentativen Maximalamplitude aus den kardialen Signaldaten;
Bestimmen, dass die repräsentative Maximalamplitude kleiner als eine Schwellenamplitude ist; und
Bestimmen, dass die Ursache des Zustandes mit niedriger atrioventrikulärer synchroner Stimulation eine niedrige atriale Signalamplitude ist, zumindest darauf basierend, dass die repräsentative Maximalamplitude kleiner als die Schwellenamplitude ist.

14. Medizinische Vorrichtung nach einem der Ansprüche 1-13, ferner umfassend einen Sensor (212) zum Abfühlen des kardialen Signals;
wobei die Steuerschaltung ferner ausgelegt ist zum:
Empfangen des kardialen Signals;
Erfassen von atrialen Ereignissignalen aus dem kardialen Signal gemäß mindestens einem Erfassungssteuerparameter;
Bestimmen des Zustandes mit niedriger atrioventrikulärer synchroner Stimulation basierend auf einem ersten Teil der Ventrikuläres-Ereignis-Daten, der einem ersten Überwachungszeitraum entspricht;
Bestimmen des Zustandes mit hoher atrioventrikulärer synchroner Stimulation basierend auf einem zweiten Teil der Ventrikuläres-Ereignis-Daten, der einem zweiten, von dem ersten Überwachungszeitraum verschiedenen Überwachungszeitraum entspricht;
Bestimmen, dass die Kriterien für die Erfassung falscher atrialer Ereignissignale erfüllt sind, zumindest darauf basierend, dass der zweite Teil der Ventrikuläres-Ereignis-Daten und/oder ein Teil der kardialen Signaldaten dem zweiten Überwachungszeitraum entsprechen; und
Einstellen des mindestens einen Erkennungssteuerparameters als Reaktion auf das Bestimmen, dass die Kriterien für die Erkennung falscher atrialer Ereignissignale erfüllt sind.

15. Medizinische Vorrichtung nach Anspruch 14, wobei die Steuerschaltung ferner ausgelegt ist zum:
Einstellen eines Zeitfensters entsprechend der passiven ventrikulären Füllung, wobei das Zeitfenster eine Endzeit hat;
Bestimmen einer erfassten atrialen Ereignissignalzeit für jeden Herzzyklus einer Vielzahl von Herzzyklen;
Bestimmen eines Anteils der erfassten atrialen Ereignissignalzeiten, die innerhalb eines Schwellenzeitintervalls der Endzeit des Zeitfensters auftreten;
Bestimmen, dass der Anteil der erfassten atrialen Ereignissignalzeiten, die innerhalb des Schwellenzeitintervalls der Endzeit des Zeitfensters auftreten, größer als ein Schwellenanteil ist; und
Bestimmen, dass die Kriterien für ein falsches atriales Ereignissignal erfüllt sind, basierend darauf, dass der Anteil der erfassten atrialen Ereignissignalzeiten, die innerhalb eines Schwellenzeitintervalls der Endzeit des Zeitfensters auftreten, größer ist als der Schwellenanteil;
und optional, wobei die Steuerschaltung ferner ausgelegt ist, um den mindestens einen Erfassungssteuerparameter einzustellen, als Reaktion auf das Bestimmen, dass die Kriterien für die Erfassung eines falschen atrialen Ereignissignals erfüllt sind, indem zumindest eine Erfassungsschwellenamplitude eines atrialen Ereignisses erhöht und/oder eine Endzeit eines Zeitfensters, das der passiven ventrikulären Füllung entspricht, erhöht wird.

## Revendications

1. Dispositif médical comprenant :
un circuit de commande (206) configuré pour :
déterminer une condition de stimulation synchrone atrioventriculaire faible sur la base de données d'événements ventriculaires ;
déterminer une cause de la condition de stimulation synchrone atrioventriculaire faible sur la base des données d'événements ventriculaires et/ou des données de signaux cardiaques ;
sélectionner au moins un réglage de paramètre de commande de fonctionnement sur la base de la cause déterminée de la condition de stimulation synchrone atrioventriculaire faible ;
fonctionner selon l'au moins un réglage de paramètre de commande de fonctionnement sélectionné ; et
un générateur d'impulsions (202) commandé par le circuit de commande pour délivrer des impulsions de stimulation ventriculaire tout en fonctionnant selon l'au moins un réglage de paramètre de commande de fonctionnement sélectionné.

2. Dispositif médical selon la revendication 1, dans lequel le circuit de commande est en outre configuré pour déterminer la condition de stimulation synchrone atrioventriculaire faible en :
déterminant, à partir des données d'événements ventriculaires, un pourcentage d'impulsions de stimulation synchrone atrioventriculaire sur un nombre total d'événements ventriculaires ; et
déterminant si le pourcentage d'impulsions de stimulation synchrone atrioventriculaire est inférieur à un pourcentage seuil.

3. Dispositif médical selon l'une quelconque des revendications 1 à 2, dans lequel le circuit de commande est en outre configuré pour déterminer la condition de stimulation synchrone atrioventriculaire faible en :
déterminant, à partir des données d'événements ventriculaires, un pourcentage d'impulsions de stimulation synchrone non atrioventriculaire sur un nombre total d'événements ventriculaires ; et
déterminant que le pourcentage d'impulsions de stimulation synchrone non atrioventriculaire est supérieur à un pourcentage seuil.

4. Dispositif médical selon l'une quelconque des revendications 1 à 3 dans lequel le réglage du paramètre de commande de fonctionnement est au moins l'un parmi :
une fréquence de stimulation inférieure ;
un incrément d'intervalle de lissage de fréquence de stimulation ;
un mode de stimulation ;
une amplitude seuil de détection d'événement atrial ;
une heure de fin de fenêtre de détection d'événement atrial ; et
une période réfractaire atriale post-ventriculaire.

5. Dispositif médical selon l'une quelconque des revendications 1 à 4, dans lequel le circuit de commande est en outre configuré pour déterminer la cause de la condition de stimulation synchrone atrioventriculaire faible en tant qu'au moins l'un d'un rythme atrial lent, d'un rythme atrial rapide et d'une faible amplitude de signal atrial.

6. Dispositif médical selon l'une quelconque des revendications 1 à 5, dans lequel le circuit de commande est en outre configuré pour :
déterminer un pourcentage d'impulsions de stimulation synchrone non atrioventriculaire à partir des données d'événements ventriculaires ;
déterminer si le pourcentage d'impulsions de stimulation synchrone non atrioventriculaire est supérieur à un pourcentage seuil ; et
déterminer la cause de la condition de stimulation synchrone atrioventriculaire faible comme étant une fréquence atriale lente sur la base du fait qu'au moins le pourcentage d'impulsions de stimulation synchrone non atrioventriculaire est supérieur au pourcentage seuil.

7. Dispositif médical selon l'une quelconque des revendications 1 à 6, dans lequel le circuit de commande est en outre configuré pour déterminer que la cause de la condition de stimulation synchrone atrioventriculaire faible est une fréquence atriale lente par au moins l'un des éléments suivants :
la détermination qu'un intervalle de temps depuis l'implantation du dispositif médical est supérieur à un intervalle de temps seuil ; et
la détermination qu'un réglage de fréquence inférieure de stimulation utilisé pour commander le générateur d'impulsions pour délivrer les impulsions de stimulation ventriculaire est plus rapide qu'une fréquence inférieure de stimulation précédente et que la fréquence inférieure de stimulation précédente est associée à un pourcentage d'impulsions de stimulation synchrone atrioventriculaire plus élevé que la condition de stimulation synchrone atrioventriculaire faible.

8. Dispositif médical selon l'une quelconque des revendications 1 à 5 comprenant en outre un capteur (212) pour détecter le signal cardiaque,
dans lequel le circuit de commande est en outre configuré pour :
recevoir le signal ;
pour chaque cycle d'une pluralité de cycles cardiaques :
régler une fenêtre temporelle correspondant à une phase de remplissage ventriculaire passif ; et
déterminer une amplitude maximale du signal cardiaque pendant la fenêtre temporelle ;
déterminer qu'une distribution des amplitudes maximales n'est pas gaussienne ; et
déterminer la cause de la condition de stimulation synchrone atrioventriculaire faible comme étant une fréquence atriale rapide sur la base d'au moins la distribution non gaussienne des amplitudes maximales.

9. Dispositif médical selon l'une quelconque des revendications 1 à 5 et 8 comprenant en outre un capteur (212) pour détecter le signal cardiaque;
dans lequel le circuit de commande est en outre configuré pour :
recevoir le signal cardiaque ;
détecter des signaux d'événements atriaux à partir du signal cardiaque ;
déterminer un temps d'événement atrial détecté pour chaque signal d'une pluralité de signaux d'événements atriaux détectés ;
déterminer quel les temps d'événements atriaux détectés répondent à des critères d'événements atriaux précoces ; et
déterminer que la cause de la condition de stimulation synchrone atrioventriculaire faible est un rythme atrial rapide sur la base du fait qu'au moins les temps d'événements atriaux détectés répondent à des critères d'événements atriaux précoces.

10. Dispositif médical selon l'une quelconque des revendications 1 à 9, dans lequel le circuit de commande est en outre configuré pour :
déterminer, à partir des données d'événements ventriculaires, des longueurs de cycles ventriculaires correspondant aux impulsions de stimulation ventriculaire synchrone non atriale délivrées par le générateur d'impulsions ;
déterminer que les longueurs de cycles ventriculaires répondent à des critères de variabilité ; et
déterminer la cause de la condition de stimulation synchrone atrioventriculaire faible comme étant une fréquence atriale rapide sur la base du fait qu'au moins les longueurs de cycles ventriculaires répondent aux critères de variabilité.

11. Dispositif médical selon l'une quelconque des revendications 1 à 10, comprenant en outre un capteur (212) pour détecter le signal cardiaque ;
dans lequel le circuit de commande est en outre configuré pour :
recevoir le signal cardiaque ;
détecter des signaux d'événements atriaux à partir du signal cardiaque ;
déterminer des amplitudes de crête du signal cardiaque correspondant aux signaux d'événements atriaux ;
déterminer qu'une amplitude représentative des amplitudes de crête est inférieure à une amplitude seuil ; et
déterminer que la cause de la condition de stimulation synchrone atrioventriculaire faible est une faible amplitude de signal atrial sur la base du fait qu'au moins l'amplitude représentative des amplitudes de crête est inférieure à une amplitude seuil.

12. Dispositif médical selon l'une quelconque des revendications 1 à 11, dans lequel le circuit de commande est en outre configuré pour :
déterminer qu'un seuil de détection d'événement atrial qui est appliqué au signal cardiaque pour détecter des signaux d'événements atriaux est inférieur à un réglage de faible amplitude ; et
déterminer que la cause de la condition de stimulation synchrone atrioventriculaire faible est une faible amplitude de signal atrial sur la base du fait qu'au moins le seuil de détection d'événement atrial est inférieur au réglage de faible amplitude.

13. Dispositif médical selon l'une quelconque des revendications 1 à 12, dans lequel le circuit de commande est en outre configuré pour :
déterminer une amplitude maximale représentative à partir des données de signal cardiaque ;
déterminer que l'amplitude maximale représentative est inférieure à une amplitude seuil ; et
déterminer que la cause de la condition de stimulation synchrone atrioventriculaire faible est une faible amplitude de signal atrial sur la base du fait qu'au moins l'amplitude maximale représentative est inférieure à l'amplitude seuil.

14. Dispositif médical selon l'une quelconque des revendications 1 à 13 comprenant en outre un capteur (212) pour détecter le signal cardiaque ;
dans lequel le circuit de commande est en outre configuré pour :
recevoir le signal cardiaque ;
détecter des signaux d'événements atriaux à partir du signal cardiaque sur la base d'au moins un paramètre de commande de détection ;
déterminer la condition de stimulation synchrone atrioventriculaire faible sur la base du fait qu'une première partie des données d'événements ventriculaires correspond à une première période de surveillance ;
déterminer une condition de stimulation synchrone atrioventriculaire élevée sur la base du fait qu'une seconde partie des données d'événements ventriculaires correspond à une seconde période de surveillance différente de la première période de surveillance ;
déterminer que des critères de détection de faux signaux d'événements atriaux sont satisfaits sur la base du fait que la seconde partie des données d'événements ventriculaires et/ou une partie des données de signaux cardiaques correspondent à la seconde période de surveillance ; et
régler le paramètre de commande de détection en réponse à la détermination du fait que les critères de détection de faux signaux d'événements atriaux sont satisfaits.

15. Dispositif médical selon la revendication 14, dans lequel le circuit de commande est en outre configuré pour :
définir une fenêtre temporelle correspondant au remplissage passif ventriculaire, la fenêtre temporelle ayant un temps de fin ;
déterminer un temps de signal d'événement atrial détecté pour chaque cycle cardiaque d'une pluralité de cycles cardiaques ;
déterminer une proportion des temps de signaux d'événements atriaux détectés qui se produisent dans un intervalle de temps seuil de l'heure de fin de la fenêtre temporelle ;
déterminer que la proportion des temps de signaux d'événements atriaux détectés qui se produisent dans l'intervalle de temps seuil de l'heure de fin de la fenêtre temporelle est supérieure à une proportion seuil ; et
déterminer que les critères de faux signaux d'événements atriaux sont remplis sur la base du fait que la proportion des temps de signaux d'événements atriaux détectés qui se produisent dans un intervalle de temps seuil de l'heure de fin de la fenêtre temporelle est supérieure à la proportion seuil ;
et éventuellement dans lequel le circuit de commande est en outre configuré pour ajuster l'au moins un paramètre de commande de détection en réponse à la détermination que les critères de détection de faux signaux d'événements atriaux sont satisfaits par une augmentation de l'amplitude seuil de détection d'événements atriaux et/ou une augmentation du temps de fin d'une fenêtre temporelle correspondant au remplissage passif ventriculaire.
